# DEMANDE DE BREVET EUROPEEN

(11) **EP 1 449 921 A2**
(43) Date de publication de la demande: **25.08.2004**
(21) Numéro de dépôt: 04075986.2
(22) Date de dépôt: 28.01.1993
(51) Int. Cl.: C12N 15/12, C12N 15/62, C12N 15/81, C12P 21/02, C12N 1/19, C07K 14/435, A61K 38/16

(54) **Nouveaux polypeptides biologiquement actifs, leur préparation et composition pharmaceutique les contenant**

(30) Priorité: 31.01.1992 FR 9201064
(62) Demande divisionnaire de: 93904129.9
(71) Demandeur: Aventis Pharma S.A., 92160 Antony (FR)
(72) Inventeur: Fleer, Reinhard, 91440 Bures-sur-Yvette (FR); Fournier, Alain, 92000 Châtenay-Malabry (FR); Guitton, Jean-Dominique, 75013 Paris (FR); Jung, Gérard, 91310 Montlhéry (FR); Yeh, Patrice, 75005 Paris (FR)
(74) Mandataire: Bassett, Richard Simon

(57) **Abrégé**

La présente invention concerne de nouveaux polypeptides biologiquement actifs, leur préparation et des compositions pharmaceutiques les contenant.

## Description

La présente invention concerne de nouveaux polypeptides biologiquement actifs, leur préparation et des compositions pharmaceutiques les contenant.

Plus particulièrement. la présente invention concerne des polypeptides recombinants essentiellement composés d'une partie active dérivée d'un polypeptide, naturel ou artificiel, ayant une activité thérapeutique, et couplé à une albumine ou à un variant de l'albumine. Il est entendu que l'activité thérapeutique des polypeptides de l'invention peut être soit directe (traitement des maladies), ou indirecte (et par exemple utilisable dans la prévention des maladies, dans la conception des vaccins, dans les techniques de l'imagerie médicale etc...).

Il est entendu dans ce qui suit que les variants de l'albumine désignent toute protéine à haute demie-vie plasmatique obtenue par modification (mutation, délétion et/ou addition) par les techniques du génie génétique d'un gène codant pour un isomorphe donné de la sérum-albumine humaine, ainsi que toute macromolécule à haute demie-vie plasmatique obtenue par modification in vitro de la protéine codée par de tels gènes. L'albumine étant très polymorphe, de nombreux variants naturels ont été identifiés et répertoriés [Weitkamp L.R. et al., Ann. Hum. Genet. 37 (1973) 219].

Le but de la présente invention est d'élaborer des protéines artificielles biologiquement actives et utilisables sur le plan pharmaceutique. En effet, de nombreux polypeptides possédant une ou plusieurs activités thérapeutiques potentielles ne peuvent être exploités pharmaceutiquement. Ceci peut avoir différentes raisons, telles que notamment leur faible stabilité in vivo, leur structure complexe ou fragile, la difficulté de les produire à une échelle industriellement acceptable, etc... De même, certains polypeptides ne donnent pas les résultats attendus in vivo en raison de problèmes d'administration, de conditionnement, de pharmacocinétique etc...

La présente invention permet de remédier à ces inconvénients. La présente invention fournit en effet de nouvelles molécules permettant une exploitation optimale sur le plan thérapeutique des propriétés biologiques de ces polypeptides. La présente invention résulte notamment de la mise en évidence qu'il est possible de coupler génétiquement toute structure active dérivée d'un polypeptide biologiquement actif à une autre structure protéique constituée d'albumine, sans en altérer lesdites propriétés biologiques. Elle résulte également de la mise en évidence par la demanderesse que la sérum-albumine humaine permet de présenter efficacement la structure active à ses sites d'interaction, et qu'elle assure une stabilité plasmatique élevée du polypeptide de l'invention. Les polypeptides de l'invention permettent ainsi de maintenir dans l'organisme une activité biologique donnée pendant un temps prolongé. Ils permettent ainsi de réduire les doses administrées et, dans certains cas, de potentialiser l'effet thérapeutique, par exemple en réduisant les effets secondaires consécutifs à une administration plus importante. Les polypeptides de l'invention permettent de plus de générer et d'utiliser des structures dérivées des polypeptides biologiquement actifs très petites et donc très spécifiques d'un effet recherché. Il est entendu que les peptides ayant une activité biologique présentant un intérêt thérapeutique peuvent également correspondre à des séquences peptidiques non naturelles, isolées par exemple à partir de banques peptidiques aléatoires. Les polypeptides de l'invention possèdent par ailleurs une répartition particulièrement avantageuse dans l'organisme, ce qui modifie leurs propriétés pharmacocinétiques et favorise le développement de leur activité biologique et leur utilisation. En outre, ils présentent également l'avantage d'être faiblement ou non-immunogéniques pour l'organisme dans lequel ils sont utilisés. Finalement, les polypeptides de l'invention peuvent être exprimés (et préférentiellement sécrétés) par des organismes recombinants, à des niveaux permettant leurs exploitation industrielle.

Un objet de la présente invention concerne donc des polypeptides comportant une partie active dérivée d'un polypeptide ayant une activité thérapeutique, couplée à une albumine ou à un variant de l'albumine.

Dans un mode de réalisation particulier, les peptides possédant une activité thérapeutique ne sont pas d'origine humaine. Par exemple on peut citer des peptides, ou leurs dérivés, possédant des propriétés potentiellement utiles dans les pathologies des compartiments sanguins et interstitiels, tels que l'hirudine, la trigramine, l'antistatine, les peptides anticoagulant des tiques (TAP), l'ariétine, l'applagine etc....

Plus particulièrement, dans les molécules de l'invention, le polypeptide ayant une activité thérapeutique est un polypeptide d'origine humaine ou un variant moléculaire. Par exemple, il peut s'agir de tout ou partie, d'un enzyme, d'un inhibiteur d'enzyme, d'un antigène, d'un anticorps, d'une hormone, d'un facteur intervenant dans le contrôle de la coagulation, d'un interféron, d'une cytokine [les interleukines, mais aussi leurs variants antagonistes naturels de leur fixation au(x) récepteur(s), les cytokines de type SIS (small induced secreted) et par exemple les protéines inflammatoires des macrophages (les MIPs), etc...], d'un facteur de croissance et/ou de différenciation [et par exemple les facteurs de croissance transformants (les TGFs), les facteurs de différenciation des cellules sanguines (érythropoiétine, M-CSF, G-CSF, GM-CSF etc..), l'insuline et les facteurs de croissance qui lui ressemblent (les IGFs), ou encore les facteurs de perméabilité cellulaire (VPF/VEGF), etc..], d'un facteur impliqué dans la génèse/résorption des tissus osseux (OIF et ostéospontine par exemple), d'un facteur impliqué dans la motilité ou la migration cellulaire [et par exemple le facteur de motilité autocrine (AMF), le facteur de stimulation de la migration (MSF), ou encore le facteur de dispersion (scatter factor/facteur de croissance des hépatocytes)], d'un facteur bactéricide ou antifongique, d'un facteur chimiotactique [et par exemple le facteur plaquettaire 4 (PF4), ou encore les peptides chemoattractants des monocytes (MCP/MCAF) ou des neutrophiles (NCAF), etc...], d'un facteur cytostatique (et par exemple les protéines qui se fixent aux galactosides), d'une molécule adhésive plasmatique (et par exemple le facteur de von Willebrand, le fibrinogène etc...) ou interstitielle (laminine, ténascine, vitronectine, etc...) ou des matrices extracellulaires, ou encore toute séquence peptidique antagoniste ou agoniste d'interactions moléculaires et/ou intercellulaires impliquées dans les pathologies des compartiments circulatoires et interstitiels et par exemple la formation des thrombus artériels et veineux, les métastases cancéreuses, l'angiogénèse tumorale, le choc inflammatoire, les maladies autoimmunes, les pathologies osseuses et ostéoarticulaires etc...

La partie active des polypeptides de l'invention peut être constituée, par exemple, par le polypeptide ayant une activité thérapeutique entier, ou par une structure dérivée de celui-ci, ou encore par un polypeptide non naturel isolé à partir d'une banque peptidique. Au sens de la présente invention, on entend par structure dérivée tout polypeptide obtenu par modification et conservant une activité thérapeutique. Par modification, on doit entendre toute mutation, substitution, délétion, addition ou modification de nature génétique et/ou chimique. De tels dérivés peuvent être générés dans des buts différents, tels que notamment celui d'augmenter l'affinité de la molécule pour ses sites de fixation, celui d'améliorer ses niveaux de production, celui d'augmenter sa résistances aux protéases, celui d'augmenter son efficacité thérapeutique ou encore de réduire ses effets secondaires, ou celui de lui conférer de nouvelles propriétés biologiques. A titre d'exemple, les polypeptides chimères de l'invention possèdent des propriétés pharmacocinétiques et une activité biologique utilisable pour la prévention ou le traitement des maladies.

Des polypeptides de l'invention particulièrement avantageux sont ceux dans lesquels la partie active présente :
(a) la structure peptidique entière ou,
(b) une structure dérivée de (a) par modification structurale (mutation, substitution addition et/ou délétion d'un ou plusieurs résidus) et possédant une activité thérapeutique.

Parmi les structures du type (b), on peut citer plus particulièrement les molécules dans lesquelles certains sites de N- ou O-glycosylation ont été modifiés ou supprimés, les molécules dans lesquelles un ou plusieurs résidus ont été substitués, ou les molécules dans lesquelles tous les résidus cystéine ont été substitués. On peut citer également des molécules obtenues à partir de (a) par délétion de régions n'intervenant pas ou peu dans l'interaction avec les sites de liaison considérés, ou exprimant une activité indésirable, et des molécules comportant par rapport à (a) des résidus supplémentaires, tels que par exemple une méthionine N-terminale et/ou un signal de sécrétion et/ou un peptide de jonction.

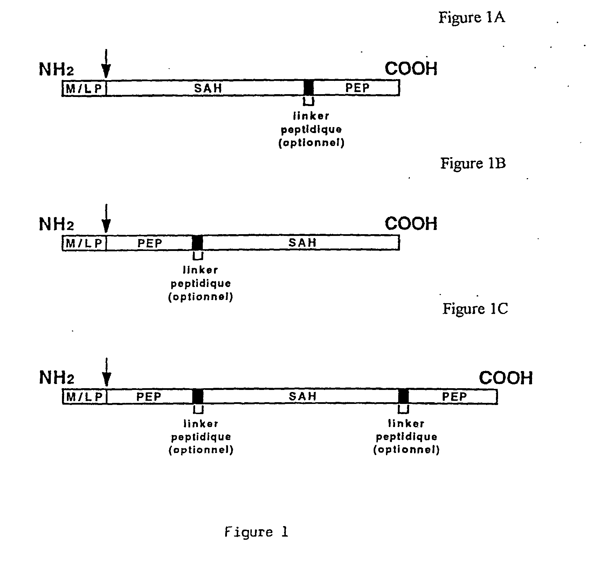

La partie active des molécules de l'invention peut être couplée soit directement soit par l'intermédiaire d'un peptide artificiel à l'albumine. De plus, elle peut constituer l'extrémité N-terminale comme l'extrémité C-terminale de la molécule. Préférentiellement, dans les molécules de l'invention, la partie active constitue la partie C-terminale de la chimère. Il est également entendu que la partie biologiquement active peut être redondante au sein de la chimère. Une représentation schématique des molécules de l'invention est donnée à la Figure 1.

Un autre objet de l'invention concerne un procédé de préparation des molécules chimères décrites ci-avant. Plus précisément, ce procédé consiste à faire exprimer par un hôte cellulaire eucaryote ou procaryote une séquence nucléotidique codant pour le polypeptide désiré, puis à récolter le polypeptide produit.

Parmi les hôtes eucaryotes utilisables dans le cadre de la présente invention, on peut citer les cellules animales, les levures, ou les champignons. En particulier, s'agissant de levures, on peut citer les levures du genre Saccharomyces, Kluyveromyces, Pichia, Schwanniomyces, ou Hansenula. S'agissant de cellules animales, on peut citer les cellules COS, CHO, C127, etc... Parmi les champignons susceptibles d'être utilisés dans la présente invention, on peut citer plus particulièrement Aspergillus ssp. ou Trichoderma ssp. Comme hôtes procaryotes, on préfère utiliser les bactéries telles que Escherichia coli, ou appartenant aux genres Corynebacterium, Bacillus, ou Streptomyces.

Les séquences nucléotidiques utilisables dans le cadre de la présente invention peuvent être préparées de différentes manières. Généralement, elles sont obtenues en assemblant en phase de lecture les séquences codant pour chacune des parties fonctionnelles du polypeptide. Celles-ci peuvent être isolées par les techniques de l'homme de l'art, et par exemple directement à partir des ARN messsagers (ARNm) cellulaires, ou par reclonage à partir d'une banque d'ADN complémentaire (ADNc), ou encore il peut s'agir de séquences nucléotidiques totalement synthétiques. Il est entendu de plus que les séquences nucléotidiques peuvent également être ultérieurement modifiées, par exemple par les techniques du génie génétique, pour obtenir des dérivés ou des variants desdites séquences.

Plus préférentiellement, dans le procédé de l'invention, la séquence nucléotidique fait partie d'une cassette d'expression comprenant une région d'initiation de la transcription (région promoteur) permettant, dans les cellules hôtes, l'expression de la séquence nucléotidique placée sous son contrôle et codant pour les polypeptides de l'invention. Cette région peut provenir de régions promoteurs de gènes fortement exprimés dans la cellule hôte utilisée, l'expression étant constitutive ou régulable. S'agissant de levures, il peut s'agir du promoteur du gène de la phosphoglycérate kinase (PGK), de la glycéraldéhyde-3-phosphate déshydrogénase (GPD), de la lactase (LAC4), des énolases (ENQ), des alcools deshydrogénases (ADH), etc... S'agissant de bactéries, il peut s'agir du promoteur des gènes droit ou gauche du bactériophage lambda (P_{L}, P_{R}), ou encore des promoteurs des gènes des opérons tryptophane (Pₜᵣₚ) ou lactose (P_{lac}). En outre, cette région de contrôle peut être modifiée, par exemple par mutagénèse in vitro, par introduction d'éléments additionnels de contrôle ou de séquences synthétiques, ou par des délétions ou des substitutions des éléments originels de contrôle. La cassette d'expression peut également comprendre une région de terminaison de la transcription fonctionnelle dans l'hôte envisagé, positionnée immédiatement en aval de la séquence nucléotidique codant pour un polypeptide de l'invention.

Dans un mode préféré, les polypeptides de l'invention résultent de l'expression dans un hôte eucaryote ou procaryote d'une séquence nucléotidique et de la sécrétion du produit d'expression de ladite séquence dans le milieu de culture. Il est en effet particulièrement avantageux de pouvoir obtenir par voie recombinante des molécules directement dans le milieu de culture. Dans ce cas, la séquence nucléotidique codant pour un polypeptide de l'invention est précédée d'une séquence "leader" (ou séquence signal) dirigeant le polypeptide naissant dans les voies de sécrétion de l'hôte utilisé. Cette séquence "leader" peut être la séquence signal naturelle du polypeptide biologiquement actif dans le cas où celui-ci est une protéine naturellement sécrétée, ou celle de la structure stabilisatrice, mais il peut également s'agir de toute autre séquence "leader" fonctionnelle, ou d'une séquence "leader" artificielle. Le choix de l'une ou l'autre de ces séquences est notamment guidé par l'hôte utilisé. Des exemples de séquences signal fonctionnelles incluent celles des gènes des phéromones sexuelles ou des toxines "killer" de levures.

En plus de la cassette d'expression, un ou plusieurs marqueurs permettant de sélectionner l'hôte recombiné peuvent être additionnés, tels que par exemple le gène URA3 de la levure S. cerevisiae, ou des gènes conférant la résistance à des antibiotiques comme la généticine (G418) ou à tout autre composé toxique comme certains ions métalliques.

L'ensemble constitué par la cassette d'expression et par le marqueur de sélection peut être introduit directement dans les cellules hôtes considérées, soit inséré préalablement dans un vecteur autoréplicatif fonctionnel. Dans le premier cas, des séquences homologues à des régions présentes dans le génôme des cellules hôtes sont préférentiellement additionnées à cet ensemble; lesdites séquences étant alors positionnées de chaque côté de la cassette d'expression et du gène de sélection de façon à augmenter la fréquence d'intégration de l'ensemble dans le génôme de l'hôte en ciblant l'intégration des séquences par recombinaison homologue. Dans le cas où la cassette d'expression est insérée dans un système réplicatif, un système de réplication préféré pour les levures du genre Kluyveromyces est dérivé du plasmide pKD1 initialement isolé de K.drosophilarum; un système préféré de réplication pour les levures du genre Saccharomyces est dérivé du plasmide 2µ de S. cerevisiae. De plus, ce plasmide d'expression peut contenir tout ou partie desdits systèmes de réplication, ou peut combiner des éléments dérivés du plasmide pKD1 aussi bien que du plasmide 2µ.

En outre, les plasmides d'expression peuvent être des vecteurs navettes entre un hôte bactérien tel que Escherichia coli et la cellule hôte choisie. Dans ce cas, une origine de réplication et un marqueur de sélection fonctionnant dans l'hôte bactérien sont requises. Il est également possible de positionner des sites de restriction entourant les séquences bactériennes et uniques sur le vecteur d'expression: ceci permet de supprimer ces séquences par coupure et religature in vitro du vecteur tronqué avant transformation des cellules hôtes, ce qui peut résulter en une augmentation du nombre de copies et en une stabilité accrue des plasmides d'expression dans lesdits hôtes. Par exemple, de tels sites de restriction peuvent correspondre aux séquences telles que 5'-GGCCNNNNNGGCC-3' SfiI) ou 5'-GCGGCCGC-3' (NotI) dans la mesure où ces sites sont extrêmement rares et généralement absents d'un vecteur d'expression.

Après construction de tels vecteurs ou cassette d'expression, ceux-ci sont introduits dans les cellules hôtes retenues selon les techniques classiques décrites dans la littérature. A cet égard, toute méthode permettant d'introduire un ADN étranger dans une cellule peut être utilisée. Il peut s'agir notamment de transformation, électroporation, conjugaison, ou toute autre technique connue de l'homme de l'art. A titre d'exemple pour les hôtes de type levure, les différentes souches de Kluyveromyces utilisées ont été transformées en traitant les cellules entières en présence d'acétate de lithium et de polyéthylène glycol, selon la technique décrite par Ito et al. [J. Bacteriol. 153 (1983) 163]. La technique de transformation décrite par Durrens et al. [Curr. Genet. 18 (1990) 7] utilisant l'éthylène glycol et le diméthylsulfoxyde a également été utilisée. Il est aussi possible de transformer les levures par électroporation, selon la méthode décrite par Karube et al. [FEBS Letters 182 (1985) 90]. Un protocole alternatif est également décrit en détail dans les exemples qui suivent.

Après sélection des cellules transformées, les cellules exprimant lesdits polypeptides sont inoculées et la récupération desdits polypeptides peut être faite, soit au cours de la croissance cellulaire pour les procédés "en continu", soit en fin de croissance pour les cultures "en lots" ("batch"). Les polypeptides qui font l'objet de la présente invention sont ensuite purifiés à partir du surnageant de culture en vue de leur caractérisation moléculaire, pharmacocinétique et biologique.

Un système d'expression préféré des polypeptides de l'invention consiste en l'utilisation des levures du genre Kluyveromyces comme cellule hôte, transformées par certains vecteurs dérivés du réplicon extrachromosomique pKD1 initialement isolé chez K. marxianus var. drosophilarum. Ces levures, et en particulier K. lactis et K. fragilis sont généralement capables de répliquer lesdits vecteurs de façon stable et possèdent en outre l'avantage d'être incluses dans la liste des organismes G.R.A.S. ("Generally Recognized As Safe"). Des levures privilégiées sont préférentiellement des souches industrielles du genre Kluyveromyces capables de répliquer de façon stable lesdits plasmides dérivés du plasmide pKD1 et dans lesquels a été inséré un marqueur de sélection ainsi qu'une cassette d'expression permettant la sécrétion à des niveaux élevés des polypeptides de l'invention.

La présente invention concerne également les séquences nucléotidiques codant pour les polypeptides chimères décrits ci-avant, ainsi que les cellules recombinantes, eucaryotes ou procaryotes, comprenant de telles séquences.

La présente invention concerne aussi l'application à titre de médicament des polypeptides selon la présente invention. Plus particulièrement, l'invention a pour objet toute composition pharmaceutique comprenant un ou plusieurs polypeptides ou séquences nucléotidiques tels que décrits ci-avant. Les séquences nucléotidiques peuvent en effet être utilisées en thérapie génique.

La présente invention sera plus complètement décrite à l'aide des exemples qui suivent, qui doivent être considérés comme illustratifs et non limitatifs.

### LISTE DES FIGURES

Les représentations des plasmides indiquées dans les Figures suivantes ne sont pas traçées à l'échelle et seuls les sites de restriction importants pour la compréhension des clonages réalisés ont été indiqués.
**Figure 1 :** Schématisation des chimères du type SAH-PEPTIDE (A), du ype PEPTIDE-SAH (B) ou PEPTIDE-SAH-PEPTIDE (C). Abréviations utilisées : M/LP, résidu méthionine initiateur de la traduction, éventuellement suivie d'une séquence signal de sécrétion; SAH, albumine mature ou un de ses variants moléculaires; PEP, peptide d'origine naturelle ou artificielle possédant une propriété thérapeutique donnée. La séquence PEP peut être présente plusieurs fois dans les molécules de type A, B ou C. La flèche noire indique l'extrémité N-terminale de la protéine mature.

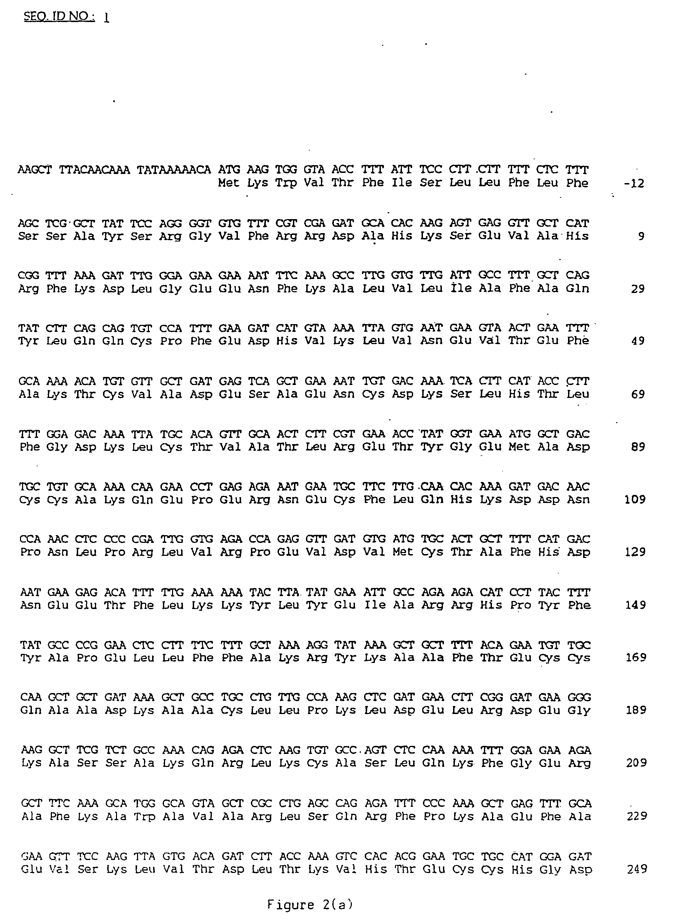

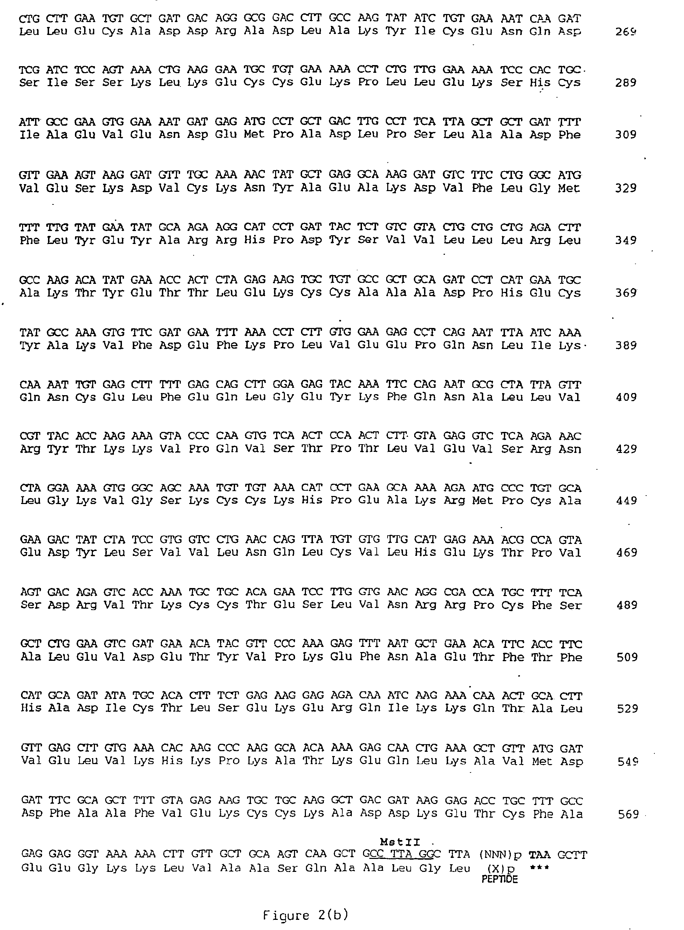

**Figure 2 :** Exemples de séquences nucléotidiques d'un fragment de restriction HindIII codant pour une protéine chimère du type prépro-SAH-PEPTIDE. Les flèches noires indiquent la fin des régions "pré" et "pro" de la SAH. Le site de restriction MstII est souligné et le codon spécifiant la terminaison de la traduction est en caractères gras.

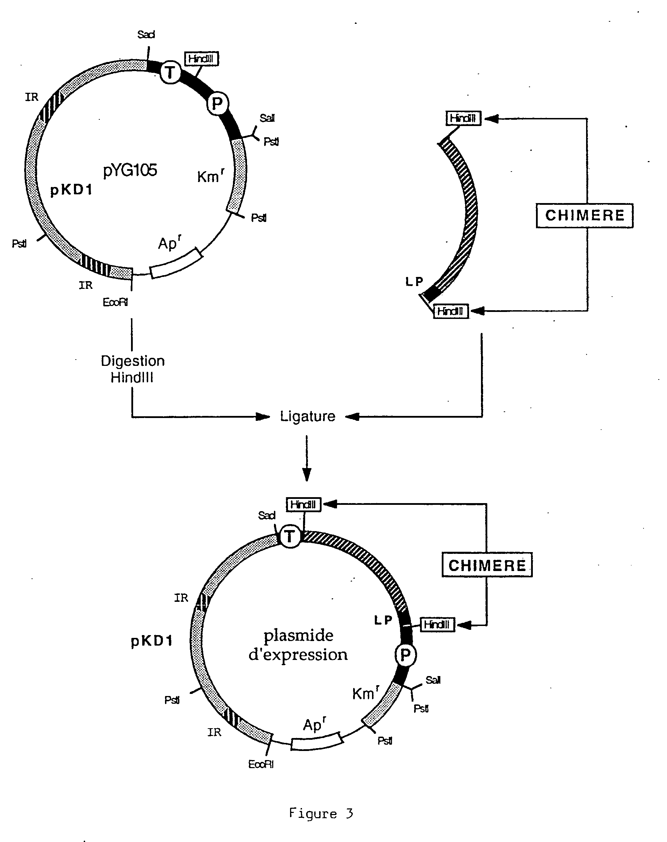

**Figure 3 :** Carte de restriction du plasmide pYG105 et stratégie générique de construction des plasmides d'expression des protéines chimères de la présente invention. Abréviations utilisées : P, promoteur transcriptionnel ; T, terminateur transcriptionnel ; IR, séquences répétées inversées du plasmide pKD1 ; LP, séquence signal de sécrétion ; Ap^{r} et Km^{r} désignent respectivement les gènes de résistance à l'ampicilline (E. coli et au G418 (levures).

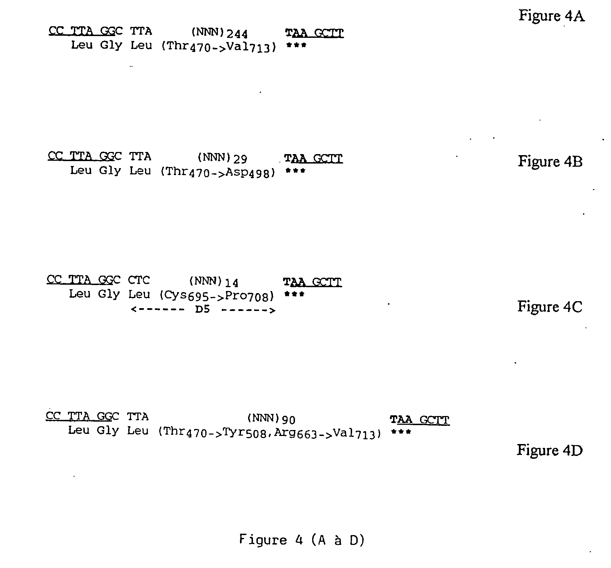

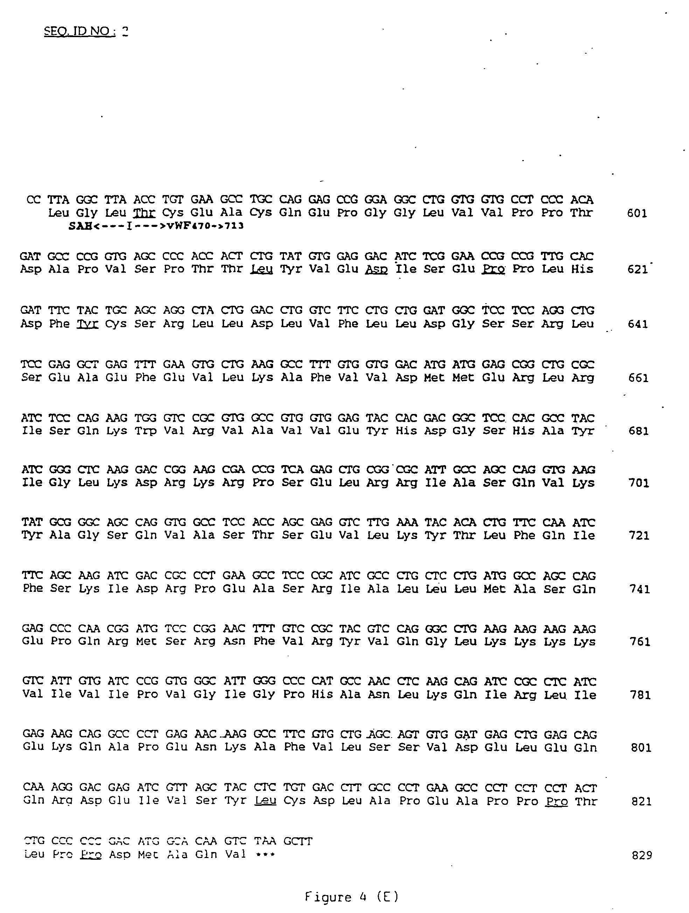

**Figure 4 :** Exemples de séquences nucléotidiques de fragments de restriction MstII-HindIII dérivés du facteur von Willebrand. Représentation de la structure des fragments MstII-HindIII des plasmides pYG1248 (panneau A), pYG1214 (panneau B), pYG1206 [panneau C, dans cette chimère particulière le résidu Leu694 du vWF est également le dernier résidu (Leu585) de la SAH] et pYG1223 (panneau D) ; la numérotation des acides aminés correspond à la numérotation du vWF mature d'après Titani et al. [Biochemistry 25 (1986) 3171-3184]. Les sites de restriction MstII et HindIII sont soulignés et le codon de terminaison de la traduction est en caractères gras. Panneau E : séquence nucléotidique du fragment de restriction MstII-HindIII du plasmide pYG1248. La numérotation des acides aminés (colonne de droite) correspond à la protéine chimère SAH-vWF470->713 mature (829 résidus). Les résidus Thr470, Leu494, Asp498, Pro502, Tyr508, Leu694, Pro704, et Pro708 du vWF mature sont soulignés.

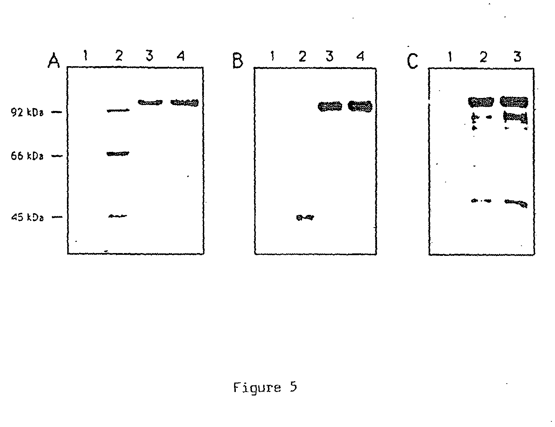

**Figure 5 :** Caractérisation du matériel sécrété après 4 jours de culture (erlenmeyers) de la souche CBS 293.91 transformée par les plasmides pYG1248 (plasmide d'expression d'une chimère du type SAH-vWF Thr470->Va17I3) et pKan707 (plasmide contrôle). Dans cette expérience les résultats des panneaux A, B, et C ont été migrés sur le même gel (SDS-PAGE 8,5 %) puis traités séparemment.
   **A**, coloration au bleu de coomassie; standard de poids moléculaire (piste 2); surnageant équivalent à 50 µl de la culture transformée par les plasmides pKan707 en milieu YPL (piste 1), ou pYG1248 en milieu YPD (piste 3) ou YPL (piste 4).
   **B**, caractérisation immunologique du matériel sécrété après utilisation d'anticorps de souris dirigés contre le vWF humain: même légende qu'en A à l'exception que des standards biotinilés de poids moléculaire ont été utilisés.
   **C**, caractérisation immunologique du matériel sécrété après utilisation d'anticorps de lapin dirigés contre l'albumine humaine: surnageant équivalent à 50 µl de la culture transformée par les plasmides pKan707 en milieu YPL (piste 1), ou pYG1248 en milieu YPD (piste 2) ou YPL (piste 3).

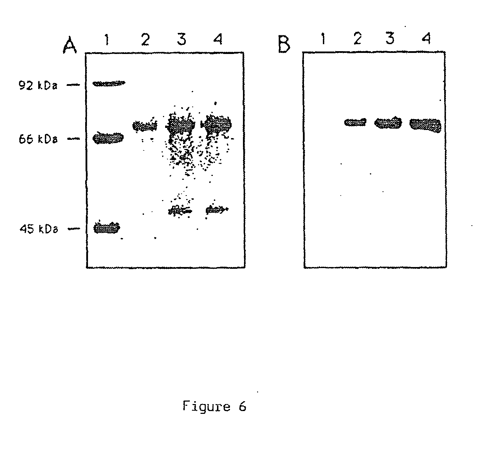

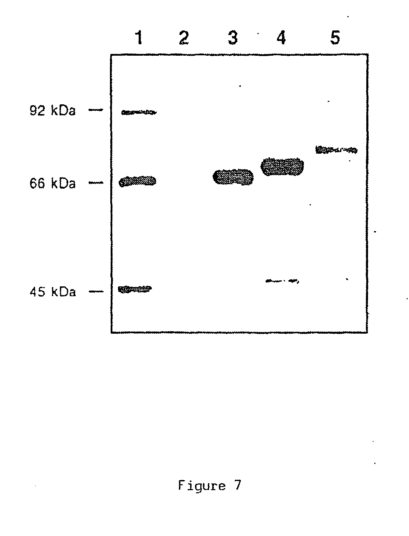

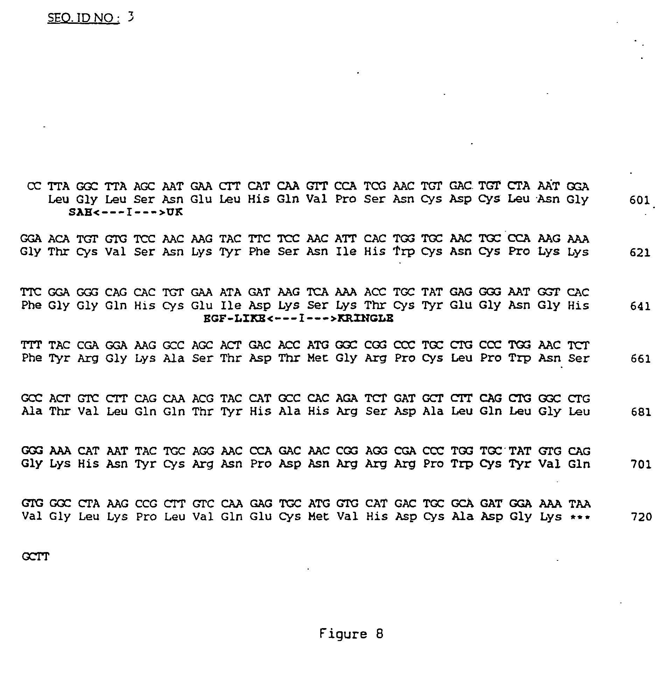

**Figure 6 :** Cinétique de sécrétion d'une chimère de l'invention par la souche CBS 293.91 transformée par le plasmide pYG1206 (SAH-vWF Leu694-Pro708).
   **A**, coloration au bleu de coomassie ; standard de poids moléculaire (piste 1) ; surnageant équivalent à 2,5 µl d'une culture "Fed Batch" en milieu YPD après 24h. (piste 2), 40h. (piste 3) ou 46h. (piste 4) de croissance.
   **B**, caractérisation immunologique du matériel sécrété après utilisation d'anticorps de souris dirigés contre le vWF humain : même légende qu'en A à l'exception que des standards biotinilés de poids moléculaire ont été utilisés.

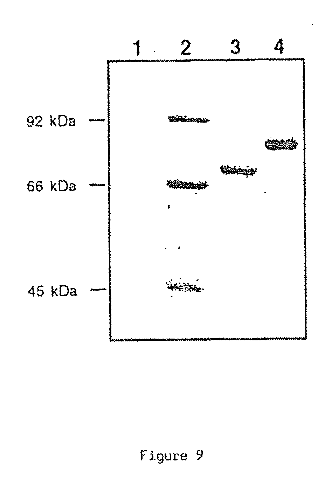

**Figure 7 :** Caractérisation du matériel sécrété par K. lactis transformé par les plasmides pKan707 (plasmide contrôle, piste 2), pYG1206 (piste 3), pYG1214 (piste 4) et pYG1223 (piste 5) ; standard de poids moléculaire (piste 1). Les dépôts correspondent à 50 µl de surnageant d'une culture stationnaire après croissance en milieu YPD, migration dans un gel à 8.5 % d'acrylamide et coloration au bleu de coomassie.
**Figure 8 :** Séquence nucléotidique du fragment de restriction MstII-HindIII du plasmide pYG1341 (SAH-UK1->135). La limite du domaine EGF-like (UK1->46) présent dans le fragment de restriction MstII-HindIII du plasmide pYG1340 est indiquée. La numérotation des acides aminés correspond à la protéine chimère SAH-UK1->135 mature (720 résidus).
**Figure 9 :** Sécrétion des chimères SAH-UK1-46 et SAH-UK1-135 par la souche CBS 293.91 respectivement transformée par les plasmides pYG1343 (SAH-UK1-46) et pYG1345 (SAH-UK1-135), après 4 jours de croissance (milieu YPL+G418). Les dépôts (équivalents à 50 µl de culture) sont migrés en gel PAGE-SDS à 8,5 % et colorés au bleu de coomassie: surnageant d'un clone transformé par les plasmides pKan707 (piste 1), pYG1343 (piste 3) ou pYG1345 (piste 4) ; standard de poids moléculaire (piste 2).

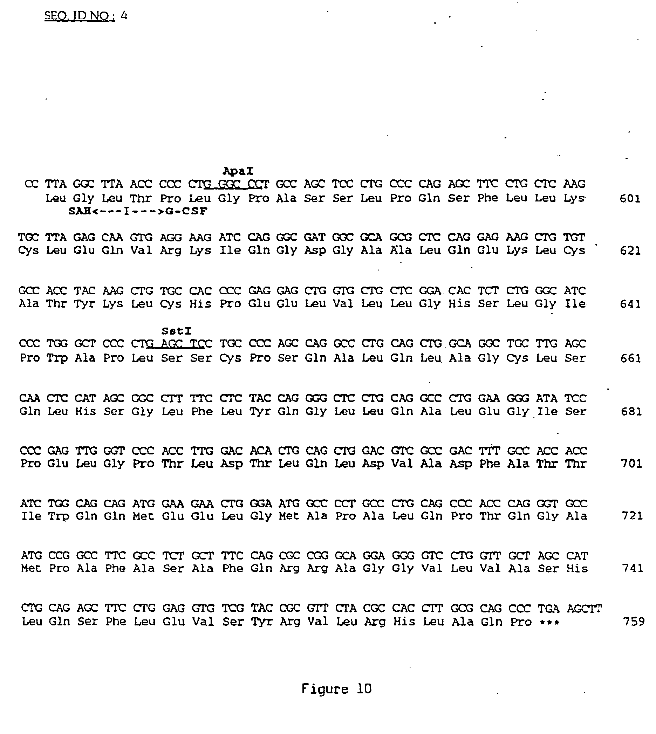

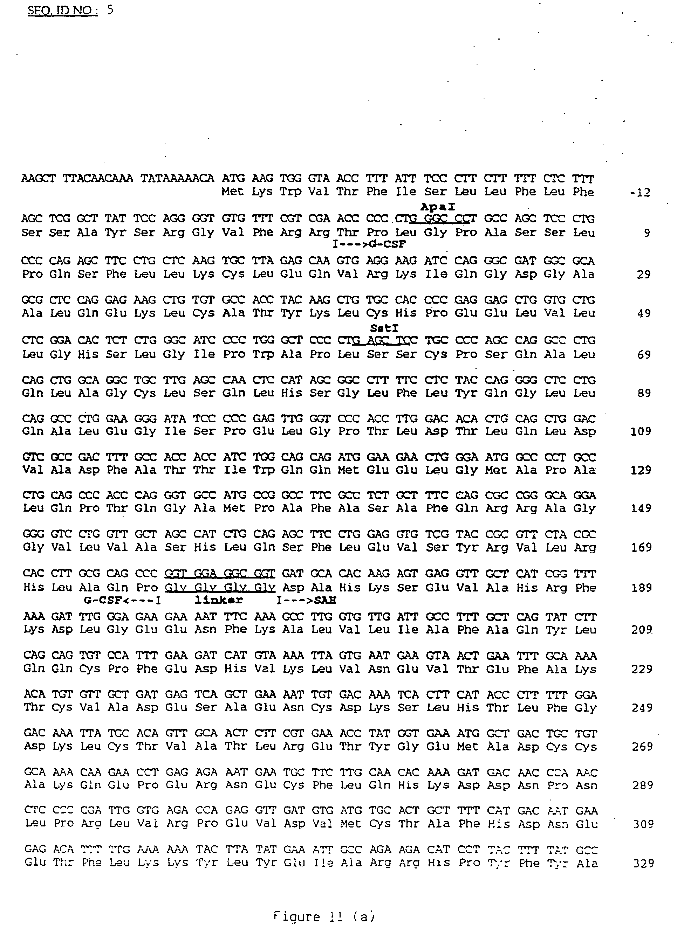

**Figure 10 :** Séquence nucléotidique du fragment de restriction MstII-HindIII du plasmide pYG1259 (SAH-G.CSF). La limite de la partie G-CSF (174 résidus) est indiquée. Les sites de restriction ApaI et SstI (SstI) sont soulignés. La numérotation des acides aminés correspond à la protéine chimère SAH-G.CSF mature (759 résidus).

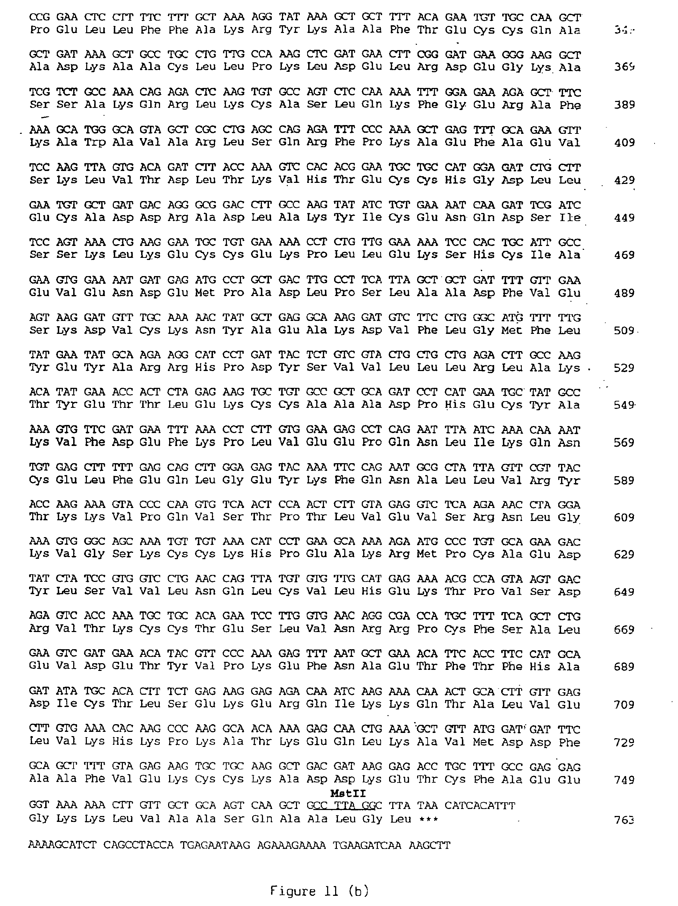

**Figure 11 :** Séquence nucléotidique du fragment de restriction HindIII du plasmide pYG1301 (chimère G.CSF-Gly₄-SAH). Les flèches noires indiquent la fin des régions "pré" et "pro" de la SAH. Les sites de restriction ApaI, SstI (SacI) et MstII sont soulignés. Les domaines G.CSF (174 résidus) et SAH (585 résidus) sont séparés par le linker synthétique GGGG. La numérotation des acides aminés correspond à la protéine chimère G.CSF-Gly4-SAH mature (763 résidus). La séquence nucléotidique comprise entre le codon de terminaison de la traduction et le site HindIII provient de l'ADN complémentaire (cDNA) de la SAH tel que décrit dans la demande de brevet EP 361 991.

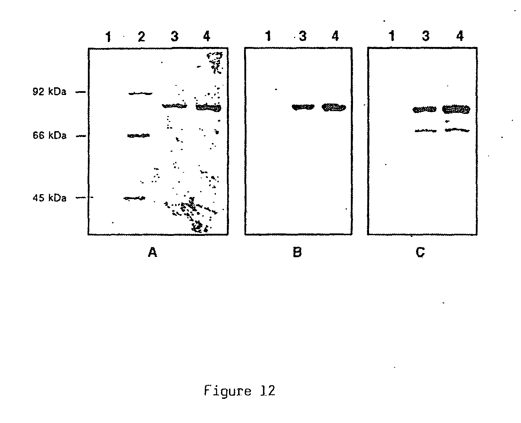

**Figure 12 :** Caractérisation du matériel sécrété après 4 jours de culture (erlenmeyers) de la souche CBS 293.91 transformée par les plasmides pYG1266 (plasmide d'expression d'une chimère du type SAH-G.CSF) et pKan707 (plasmide contrôle). Dans cette expérience les résultats des panneaux A, B, et C ont été migrés sur le même gel (SDS-PAGE 8,5 %) puis traités séparemment.
   **A**, coloration au bleu de coomassie; standard de poids moléculaire (piste 2) ; surnageant équivalent à 100 µl de la culture transformée par les plasmides pKan707 en milieu YPL (piste 1), ou pYG1266 en milieu YPD (piste 3) ou YPL (piste 4).
   **B**, caractérisation immimologique du matériel sécrété après utilisation d'anticorps primaires dirigés contre le G-CSF humain : même légende qu'en A.
   **C**, caractérisation immunologique du matériel sécrété après utilisation d'anticorps primaires dirigés contre l'albumine humaine : même légende qu'en A.

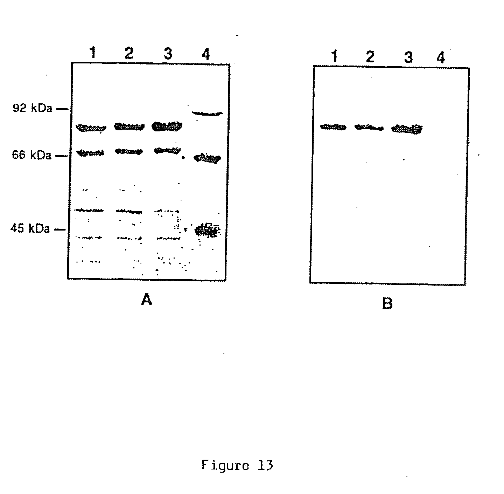

**Figure 13 :** Caractérisation du matériel sécrété après 4 jours de culture (erlenmeyers en milieu YPD) de la souche CBS 293.91 transformée par les plasmides pYG1267 (chimère SAH-G.CSF), pYG1303 (chimère G.CSF-Gly₄-SAH) et pYG1352 (chimère SAH-Gly₄-G.CSF) après migration sur gel SDS-PAGE 8,5 %.
   **A**, coloration au bleu de coomassie ; surnageant équivalent à 100 µl de la culture transformée par les plasmides pYG1303 (piste 1), pYG1267 (piste 2) ou pYG1352 (piste 3); standard de poids moléculaire (piste 4).
   **B**, caractérisation immunologique du matériel sécrété après utilisation d'anticorps primaires dirigés contre le G-CSF humain : même légende qu'en A.

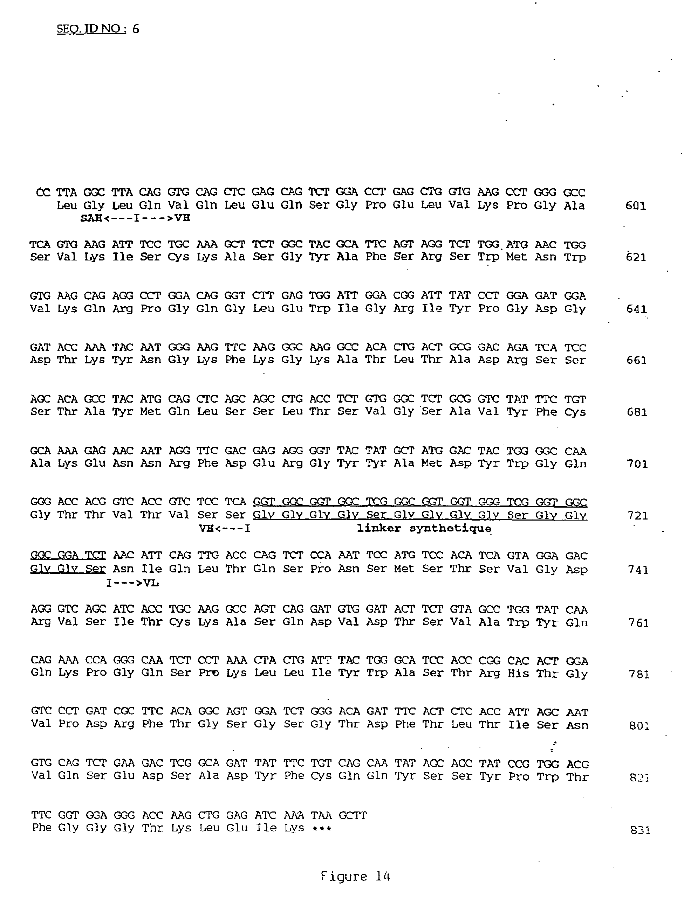

**Figure 14 :** Séquence nucléotidique du fragment de restriction MstII-HindIII du plasmide pYG1382 (SAH-Fv'). Les domaines VH (124 résidus) et VL (107 résidus) du fragment Fv' sont séparés par le linker synthétique (GGGGS)ₓ₃. La numérotation des acides aminés correspond à la protéine chimère SAH-Fv' mature (831 résidus).

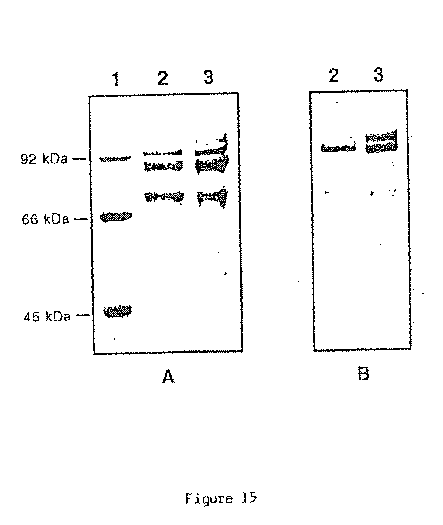

**Figure 15 :** Sécrétion de la chimère SAH-Fv' par la souche CBS 293.91 transformée par le plasmide pYG1383 (LAC4) après 4 jours de croissance en erlenmeyers à 28°C en milieu YPD (piste 2), ou YPL (piste 3) ; standard de poids moléculaire (piste 1). Les dépôts, équivalents à 200 µl de culture (précipitation à l'éthanol), sont migrés en gel PAGE-SDS (8,5 %).
   **A**, : coloration du gel au bleu de coomassie.
   **B**, : caractérisation immunologique du matériel sécrété après utilisation d'anticorps primaires dirigés contre la SAH.

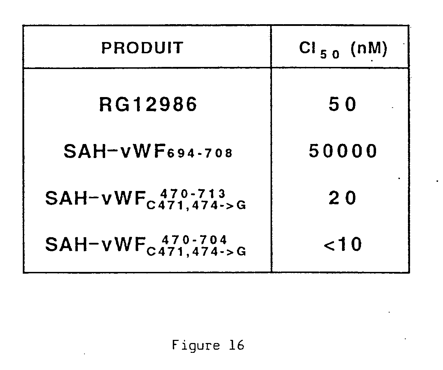

**Figure 16 :** Dosage de l'activité antagoniste in vitro de l'agglutination des plaquettes humaines fixées au formaldéhyde : CI50 des hybrides SAH-vWF694-708, [SAH-vWF470-713 C471G, C474G] et [SAH-vWF470-704 C471G, C474G] relativement à l'étalon RG12986. La détermination de l'inhibition dose-dépendante de l'agglutination plaquettaire est réalisée selon la méthode décrite par C. Prior et al. [Bio/Technology (1992) 10 66] en utilisant un agrégamètre enregistrant les variations de la transmission optique sous agitation à 37°C en présence de vWF humain, de botrocétine (8,2 mg/ml) et du produit à tester à différentes dilutions. La concentration du produit qui permet d'inhiber de moitié l'agglutination contrôle (en l'absence de produit) est alors déterminée (CI50).

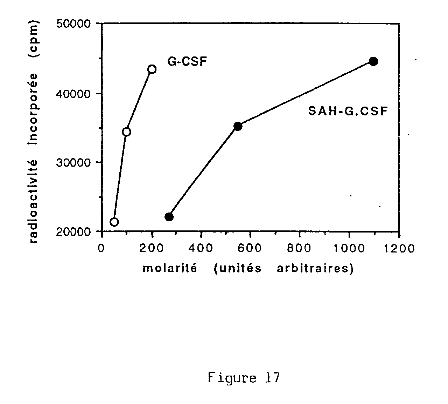

**Figure 17 :** Activité sur la prolifération cellulaire in vitro de la lignée murine NFS60. La radioactivité (³H-thymidine) incorporée dans les noyaux cellulaires après 6 heures d'incubation est représentée en ordonnée (cpm) ; la quantité de produit indiquée en abscisse est exprimée en molarité (unités arbitraires).

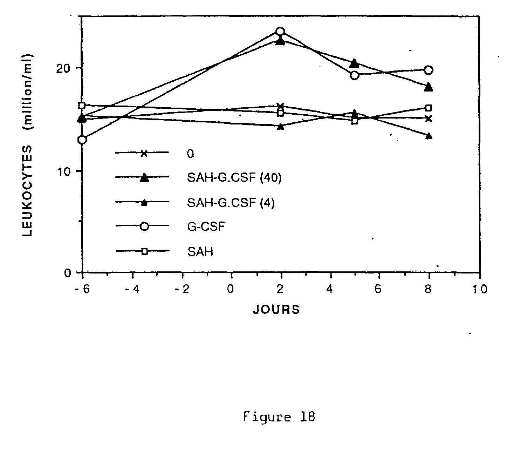

**Figure 18 :** Activité sur la granulopoièse in vivo chez le rat. Le nombre de neutrophiles (moyenne de 7 animaux) est indiquée en ordonnée en fonction du temps. Les produits testés sont la chimère SAH-G.CSF (pYG1266, 4 ou 40 mg/rat/jour), le G-CSF référence (10 mg/rat/jour), la SAH recombinante purifiée à partir de surnageant de Kluyveromyces lactis (SAH, 30 mg/rat/jour, cf. EP 361 991), ou du sérum physiologique.

### EXEMPLES

### TECHNIQUES GENERALES DE CLONAGE

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982 ; Ausubel F.M. et al. (eds), "Current Protocols in Molecular Biology", John Wiley & Sons, New York, 1987].

Les enzymes de restriction ont été fournies par New England Biolabs (Biolabs), Bethesda Research Laboratories (BRL) ou Amersham et sont utilisées selon les recommandations des fournisseurs.

Les plasmides de type pBR322, pUC et les phages de la série M13 sont d'origine commerciale (Bethesda Research Laboratories).

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes est effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques est effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] est effectuée en utilisant un "DNA thermal cycler" (Perkin Elmer Cetus) selon les spécifications du fabricant.

La vérification des séquences nucléotidiques est effectuée par la méthode développée par Sanger et al. [Proc. Natl. Acad. Sci. USA, 74 (1977) 5463-5467] en utilisant le kit distribué par Amersham.

Les transformations de K. lactis avec l'ADN des plasmides d'expression des protéines de la présente invention sont effectuées par toute technique connue de l'homme de l'art, et dont un exemple est donné dans le texte.

Sauf indication contraire, les souches bactériennes utilisées sont E. coli MC1060 (lacIPOZYA, X74, galU, galK, StrA^{r}), ou E. coli TG1 (lac, proA,B, supE, thi, hsdD5 / FtraD36, proA⁺B⁺, lacI^{q}, lacZ, M15).

Les souches de levures utilisées appartiennent aux levures bourgeonnantes et plus particulièrement aux levures du genre Kluyveromyces. Les souche K. lactis MW98-8C (a, uraA, arg, lys, K⁺, pKD1°) et K. lactis CBS 293.91 ont été particulièrement utilisées; un échantillon de la souche MW98-8C a été déposé le 16 Septembre 1988 au Centraalbureau voor Schimmelkulturen (CBS) à Baam (Pays Bas) où il a été enregistré sous le numéro CBS 579.88.

Une souche bactérienne (E. coli) transformée avec le plasmide pET-8c52K a été déposée le 17 Avril 1990 auprès de l'American Type Culture Collection sous le numéro ATCC 68306.

Les souches de levures transformées par les plasmides d'expression codant pour les protéines de la présente invention sont cultivées en erlenmeyers ou en fermenteurs pilotes de 21 (SETRIC, France) à 28°C en milieu riche (YPD: 1% yeast extract, 2% Bactopeptone, 2% glucose; ou YPL: 1% yeast extract, 2% Bactopeptone, 2% lactose) sous agitation constante.

### EXEMPLE 1 : COUPLAGE EN C-TERMINAL DE LA SAH

Le plasmide pYG404 est décrit dans la demande de brevet EP 361 991. Ce plasmide comporte un fragment de restriction HindIII codant pour le gène de la prépro-SAH précédé des 21 nucléotides naturellement présents immédiatement en amont de l'ATG initiateur de traduction du gène PGK de S. cerevisiae. La séquence nucléotidique de ce fragment de restriction est incluse dans celle de la Figure 2. Le site MstII localisé dans la séquence codante, à trois résidus du codon spécifiant la fin de traduction est particulièrement utile comme site de clonage d'un peptide biologiquement actif que l'on désire coupler en phase traductionnelle en C-terrninal de la SAH. Dans un mode de réalisation particulier, il est utile d'utiliser des peptides dont la séquence est codée par un fragment de restriction MstII-HindIII du type : 5'-CCTTAGGCTTA [3xN]ₚ TAAGCTT-3', la séquence codant le peptide (p résidus) biologiquement actif est [3xN]ₚ). La ligature de ce fragment avec le fragment de restriction HindIII-MstII correspondant à la totalité du gène codant pour la SAH à l'exception des trois acides aminés les plus C-terminaux (résidus leucine-glycine-leucine) génère un fragment de restriction HindIII comportant un gène hybride codant pour une protéine chimère du type SAH-PEPTIDE (Figure 1, panneau A), immédiatement précédée de la région d'exportation "prépro" de la SAH. Dans un autre mode de réalisation, le peptide biologiquement actif peut être présent plus d'une fois dans la chimère.

### EXEMPLE 2 : COUPLAGE EN N-TERMINAL DE LA SAH

Dans un mode réalisation particulier, les techniques combinées de mutagénèse dirigée et d'amplification PCR permettent de construire des gènes hybrides codant pour une protéine chimère résultant du couplage traductionnel entre un peptide signal (et par exemple la région prépro de la SAH), une séquence incluant le peptide biologiquement actif et la forme mature de la SAH ou un de ses variants moléculaires. Ces gènes hybrides sont préférentiellement bordés en 5' de l'ATG initiateur de traduction et en 3' du codon de fin de traduction par des sites de restriction HindIII et codent pour des protéines chimères du type PEPTIDE-SAH (Figure 1, panneau B). Dans un mode réalisation encore plus particulier, le peptide biologiquement actif peut être présent plus d'une fois dans la chimère.

### EXEMPLE 3 : COUPLAGE EN N- ET C-TERMINAL DE LA SAH

Les techniques combinées de mutagénèse dirigée et d'amplification PCR décrites dans les exemples 1 et 2 permettent de construire des gènes hybrides codant pour une protéine chimère résultant du couplage traductionnel entre la forme mature de la SAH, ou un de ses variants moléculaires, et un peptide biologiquement actif couplé aux extrémités N- et C-terminales de la SAH. Ces gènes hybrides sont préférentiellement bordés en 5' de l'ATG initiateur de traduction et en 3' du codon de fin de traduction par des sites de restriction HindIII et codent pour des protéines chimères du type PEPTIDE-SAH-PEPTIDE (Figure 1, panneau C), immédiatement précédées de la région d'exportation "prépro" de la SAH. Dans un mode réalisation encore plus particulier, le peptide biologiquement actif peut être présent plus d'une fois dans la chimère.

### EXEMPLE 4 : PLASMIDES D'EXPRESSION

Les protéines chimères des exemples précédents peuvent être exprimées dans les levures à partir de promoteurs fonctionnels, régulables ou constitutifs, tels que, par exemple, ceux présents dans les plasmides pYG105 (promoteur LAC4 de Kluyveromyces lactis), pYG106 (promoteur PGK de Saccharomyces cerevisiae), pYG536 (promoteur PHO5 de S.cerevisiae), ou des promoteur hybrides tels que ceux décrits dans la demande de brevet EP 361 991. Les plasmides pYG105 et pYG106 sont ici particulièrement utiles car ils permettent l'expression des gènes codés par les fragments de restriction HindIII tel que décrits dans les exemples précédents et clonés dans le site HindIII et dans l'orientation productive (définie comme l'orientation qui place la région "prépro" de l'albumine de façon proximale par rapport au promoteur de transcription), à partir de promoteurs fonctionnels chez K.lactis, régulables (pYG105) ou constitutifs (pYG106). Le plasmide pYG105 correspond au plasmide pKan707 décrit dans la demande de brevet EP 361 991 dans lequel le site de restriction HindIII unique et localisé dans le gène de résistance à la généticine (G418) a été détruit par mutagénèse dirigée tout en conservant une protéine inchangée (oligodeoxynucleotide 5'-GAAATGCATAAGCTCTTGCCATTCTCACCG-3'). Le fragment SalI-SacI codant pour le gène URA3 du plasmide muté a été ensuite remplacé par un fragment de restriction SalI-SacI comportant une cassette d'expression constituée du promoteur LAC4 de K. lactis (sous la forme d'un fragment SalI-HindIII) et du terminateur du gène PGK de S. cerevisiae (sous la forme d'un fragment HindIII-SacI). Le plasmide pYG105 est mitotiquement très stable chez les levures Kluyveromyces et une carte de restriction en est donnée à la Figure 3. Les plasmides pYG105 et pYG106 ne diffèrent entre eux que par la nature du promoteur de transcription encodé par le fragment SalI-HindIII.

### EXEMPLE 5 : TRANSFORMATION DES LEVURES

La transformation des levures appartenant au genre Kluyveromyces, et en particulier les souches MW98-8C et CBS 293.91 de K. lactis, s'effectue par exemple par la technique de traitement des cellules entières par de l'acétate de lithium [Ito H. et al., J. Bacteriol. 153 (1983) 163-168], adaptée comme suit. La croissance des cellules se fait à 28°C dans 50 ml de milieu YPD, avec agitation et jusqu'à une densité optique à 600 nm (DO₆₀₀) comprise entre 0,6 et 0,8; les cellules sont récoltées par centrifugation à faible vitesse, lavées dans une solution stérile de TE (10 mM Tris HCl pH 7,4; 1 mM EDTA), resuspendues dans 3-4 ml d'acétate-lithium (0,1 M dans du TE) pour obtenir une densité cellulaire d'environ 2 x 10⁸ cellules/ml, puis incubées à 30°C pendant 1 heure sous agitation modérée. Des aliquotes de 0,1 ml de la suspension résultante de cellules compétentes sont incubés à 30°C pendant 1 heure en présence d'ADN et à une concentration finale de 35% de polyéthylène glycol (PEG₄₀₀₀, Sigma). Après un choc thermique de 5 minutes à 42°C, les cellules sont lavées 2 fois, resuspendues dans 0,2 ml d'eau stérile et incubées 16 heures à 28°C dans 2 ml de milieu YPD pour permettre l'expression phénotypique du gène de résistance au G418 exprimé sous contrôle du promoteur Pₖ₁ (cf. EP 361 991); 200 µl de la suspension cellulaire sont ensuite étalés sur boites YPD sélectives (G418, 200 µg/ml). Les boites sont mises à incuber à 28°C et les transformants apparaissent après 2 à 3 jours de croissance cellulaire.

### EXEMPLE 6 : SECRETION DES CHIMERES

Après sélection sur milieu riche supplémenté en G418 les clones recombinants sont testés pour leur capacité à sécréter la forme mature des protéines chimères. Quelques clones correspondant à la souche CBS 293.91 ou MW98-8C transformée par les plasmides d'expression des chimères entre la SAH et la partie biologiquement active sont mis à incuber en milieu YPD ou YPL à 28°C. Les surnageants cellulaires sont récupérés par centrifugation quand les cellules atteignent la phase stationnaire de croissance, éventuellement concentrés 10 fois par précipitation pendant 30 minutes à -20°C dans une concentration finale de 60% d'éthanol, puis testés après électrophorèse en gel SDS-PAGE à 8.5%, soit directement par coloration du gel par du bleu de coomassie, soit après immunoblot en utilisant des anticorps primaires dirigés contre la partie biologiquement active ou un sérum polyclonal de lapin dirigé contre la SAH. Lors des expériences de détection immunologique, le filtre de nitrocellulose est d'abord incubé en présence des anticorps primaires spécifiques, lavé plusieurs fois, incubé en présence d'anticorps de chèvre dirigés contre les anticorps primaires, puis incubé en présence d'un complexe avidine-péroxydase en utilisant le "kit ABC" distribué par Vectastain (Biosys S.A., Compiègne, France). La réaction immunologique est ensuite révélée par addition de diamino-3,3' benzidine tetrachlorydrate (Prolabo) en présence d'eau oxygénée, selon les recommandations du fabricant.

### EXEMPLE 7 : CHIMERES DERIVEES DU FACTEUR VON WILLEBRAND

### E.7.1. Fragments antagonistes de la fixation du vWF aux plaquettes.

### E.7.1.1. Résidus Thr470-Va1713 du vWF.

Le plasmide pET-8c52K comporte un fragment du cDNA du vWF codant pour les résidus 445 à 733 du vWF humain et inclus donc plusieurs déterminants cruciaux de l'interaction entre le vWF et les plaquettes d'une part, et certains éléments de la membrane basale et du tissu sous-endothelial d'autre part, et notamment les peptides G10 et D5 antagonistes de l'interaction entre vWF et GP1b [Mori H. et al., J. Biol. Chem. 263 (1988) 17901-17904]. Cette séquence peptidique est identique à la séquence correspondante décrite par Titani et al. [Biochemistry 25 (1986) 3171-3184]. L'amplification de ces déterminants génétiques peut être réalisée à partir du plasmide pET-8c52K, par exemple par la technique d'amplification PCR, en utilisant comme amorce des oligodéoxynucléotides codant pour des résidus contigus localisés de part et d'autres de la séquence à amplifier. Les fragments amplifiés sont ensuite clonés dans des vecteurs du type M13 en vue de leur vérification par séquençage en utilisant soit les amorces universelles situées de part et d'autre du multisite de clonage, soit des oligodéoxynucléotides spécifiques de la région amplifiée du gène du vWF dont la séquence de plusieurs isomorphes est connue [Sadler J.E. et al., Proc. Natl. Acad. Sci. 82 (1985) 6394-6398; Verweij C.L. et al., EMBO J. 5 (1986) 1839-1847; Shelton-Inloes B.B. et al., Biochemistry 25 (1986) 3164-3171; Bonthron D. et al., Nucleic Acids Res. 17 (1986) 7125-7127]. Ainsi, l'amplification PCR du plasmide pET-8c52K avec les oligodéoxynucléotides 5'-CCCGGGATCCCTTAGGCTTAACCTGTGAAGCCTGC-3' (Sq1969, le site MstII est souligné) et 5'-CCCGGGATCCAAGCTTAGACTTGTGCCATGTCG-3' (Sq2029, le site HindIII est souligné) génère un fragment de restriction MstII-HindIII incluant les résidus Thr470 à Val713 du vWF (Figure 4, panneau E). La ligature de ce fragment avec le fragment de restriction HindIII-MstII correspondant à la totalité du gène codant pour la SAH à l'exception des trois acides aminés les plus C-terminaux (cf. Figure 2) génère un fragment de restriction HindIII comportant un gène hybride codant pour une protéine chimère du type SAH-PEPTIDE (Figure 1, panneau A), immédiatement précédée de la région d'exportation "prépro" de la SAH. Ce fragment de restriction est cloné dans l'orientation productive et dans le site HindIII du plasmide pYG105, ce qui génère le plasmide d'expression pYG1248 (SAH-vWF470-713).

### E.7.1.2. Variants moléculaires:

Dans un autre mode de réalisation, le site de liaison du vWF est un peptide incluant les résidus Thr470 à Asp498 du vWF mature. Cette séquence inclus le peptide G10 (Cys474-Pro488) décrit par Mori et al. [J. Biol. Chem. 263 (1988) 17901-17904] et capable d'antagoniser l'interaction du vWF humain à la GP1b des plaquettes humaines. La séquence correspondant au peptide G10 est d'abord incluse dans un fragment de restriction MstII-HindIII (Figure 4, panneau B), par exemple par amplification PCR du plasmide pET-8c52K avec les oligodéoxynucléotides Sq1969 et 5'-CCCGGGATCCAAGCTTAGTCCTCCACATACAG-3' (Sq1970, le site HindIII est souligné), ce qui génère un fragment de restriction MstII-HindIII incluant le peptide G10, et dont la séquence est: 5'-CCTTAGGCTTAACCTGTGAAGCCTGCCAGGAGCCGGGAGGCCTGGTGGTGCCTCCCACAGATGCCCCGGTGAGCCCCACCACTCTGTATGTGGAGGACTAAGCTT-3' (la séquence codant pour le peptide G10 est en caractères gras). La ligature de ce fragment avec le fragment de restriction HindIII-MstII correspondant à la totalité du gène codant pour la SAH à l'exception des trois acides aminés les plus C-terminaux (cf. Figure 2) génère un fragment de restriction HindIII comportant un gène hybride codant pour une protéine chimère du type SAH-PEPTIDE (Figure 1, panneau A), immédiatement précédée de la région d'exportation "prépro" de la SAH. Ce fragment de restriction est cloné dans l'orientation productive dans le site HindIII du plasmide pYG105, ce qui génère le plasmide d'expression pYG1214.

Dans un autre mode de réalisation, le site de liaison du vWF à la GP1b est directement conçu à l'aide d'oligodéoxynucléotides synthétiques, et par exemple les oligodéoxynucléotides 5'-TTAGGCCTCTGTGACCTTGCCCCTGAAGCCCCTCCTCCTACTCTGCCCCCCTAAGCTTA-3' et 5'-GATCTAAGCTTAGGGGGGCAGAGTAGGAGGAGGGGCTTCAGGGGCAAGGTCACAGAGGCC-3'. Ces oligodéoxynucléotides forment en s'appariant un fragment de restriction MstII-BglII incluant le fragment MstII-HindIII (Figure 4, panneau C) correspondant au peptide D5 défini par les résidus Leu694 à Pro708 du vWF. La ligature du fragment MstII-HindIII avec le fragment de restriction HindIII-MstII correspondant à la totalité du gène codant pour la SAH à l'exception des trois acides aminés les plus C-terminaux (cf. Figure 2) génère un fragment de restriction HindIII comportant un gène hybride codant pour une protéine chimère du type SAH-PEPTIDE (Figure 1, panneau A), immédiatement précédée de la région d'exportation "prépro" de la SAH. Ce fragment de restriction est cloné dans l'orientation productive dans le site HindIII du plasmide pYG105, ce qui génère le plasmide d'expression pYG1206.

Des variants utiles du plasmide pET-8c52K sont délétés par mutagénèse dirigée entre les peptides G10 et D5, par exemple des sites de fixation au collagène, et/ou à l'héparine, et/ou à la botrocétine , et/ou aux sulfatides et/ou à la ristocétine. Un exemple est le plasmide pMMB9 délété par mutagénèse dirigée entre les résidus Cys509 et Ile662. L'amplification PCR de ce plasmide avec les oligodéoxynucléotides Sq1969 et Sq2029 génère un fragment de restriction MstII-HindIII (Figure 4, panneau D) incluant les résidus Thr470 à Tyr508 et Arg663 à Val713 et en particulier les peptides G10 et D5 du vWF et délété en particulier de son site de fixation au collagène localisé entre les résidus Glu542 et Met622 [Roth G.J. et al. Biochemistry 25 (1986) 8357-8361]. La ligature de ce fragment avec le fragment de restriction HindIII-MstII correspondant à la totalité du gène codant pour la SAH à l'exception des trois acides aminés les plus C-terminaux (cf. Figure 2) génère un fragment de restriction HindIII comportant un gène hybride codant pour une protéine chimère du type SAH-PEPTIDE (Figure 1, panneau A), immédiatement précédée de la région d'exportation "prépro" de la SAH. Ce fragment de restriction est cloné dans l'orientation productive dans le site HindIII du plasmide pYG105, ce qui génère le plasmide d'expression pYG1223.

Dans d'autres modes de réalisation, l'utilisation des techniques combinées de mutagénèse dirigée et d'amplification PCR permet de générer à volonté des variants du fragment de restriction MstII-HindIII du panneau A de la Figure 4 mais délétés d'un ou plusieurs sites de fixation aux sulfatides et/ou à la botrocétine et/ou à l'héparine et/ou au collagène, et/ou substitué par tout résidu impliqué dans l'émergence de pathologies de type IIB associée au vWF.

Dans d'autres variants utiles du plasmide pET-8c52K des mutations sont introduites, par exemple par mutagénèse dirigée, pour remplacer ou supprimer tout ou partie de l'ensemble des cystéines présentes aux positions 471, 474, 509 et 695 du vWF humain. Des exemples particuliers sont les plasmides p5E et p7E dans lesquels les cystéines présentes aux positions 471 et 474 d'une part et aux positions 471, 474, 509 et 695 d'autre part ont été respectivement remplacés par des résidus glycine. L'amplification PCR de ces plasmides avec les oligodéoxynucléotides Sq2149 (5'-CCCGGGATCCCTTAGGCTTAACCGGTGAAGCCGGC-3', le site MstII est souligné) et Sq2029 permet de générer des fragments de restriction MstII-HindIII incluant les résidus Thr470 à Val713 du vWF naturel à l'exception qu'au moins les résidus cystéine aux positions 471 et 474 ont été mutés en des résidus glycine. La ligature de ces fragments avec le fragment de restriction HindIII-MstII correspondant à la totalité du gène codant pour la SAH à l'exception des trois acides aminés les plus C-terminaux (cf. Figure 2) génère un fragment de restriction HindIII comportant un gène hybride codant pour une protéine chimère du type SAH-PEPTIDE (Figure 1, panneau A), immédiatement précédée de la région d'exportation "prépro" de la SAH. Ces fragments de restriction sont clonés dans l'orientation productive dans le site HindIII du plasmide pYG105, ce qui génère les plasmides d'expression pYG1283 (chimère SAH-vWF470-713, C471G, C474G) et pYG1279 (chimère SAH-vWF470-713, C471G, C474G, C509G, C695G).

D'autres mutations particulièrement utiles concernent au moins un résidu impliqué dans des pathologies de type IIB associées au vWF (augmentation de l'affinité intrinsèque du vWF pour la GP1b), comme les résidus Arg543, Arg545, Trp550, Val551, Val553, Pro574 ou Arg578 par exemple. Les techniques de recombinaison génétique in vitro permettent également d'introduire à volonté un ou des résidus supplémentaires dans la séquence du vWF et par exemple une méthionine surnuméraire entre les positions Asp539 et Glu542.

### E.7.2. Fragments antagonistes de la fixation du vWF au sous endothélium.

Dans un mode de réalisation particulier, les sites de liaison du vWF aux composants du tissu sous-endothélial, et par exemple du collagène, sont générés par amplication PCR du plasmide pET-8c52K, par exemple avec les oligodéoxynucléotides Sq2258 (5'-GGATCCTTAGGGCTG-TGCAGCAGGCTACTGGACCTGGTC-3', le site MstII est souligné) et Sq2259 (5'GAATTCAAGCTTAACAGAGGTAGCTAACGATCTCGTCCC-3', le site HindIII est souligné), ce qui génère un fragment de restriction MstII-HindIII codant pour les résidus Cys509 à Cys695 du vWF naturel. Des variants moléculaires de délétion ou modifiés sont également générés qui comportent toute combinaison souhaitable entre les sites de fixation du vWF aux sulfatides et/ou à la botrocétine et/ou à l'héparine et/ou au collagène et/ou tout résidu responsable d'une modification de l'affinité du vWF pour la GP1b (pathologies de type Il associée au vWF). Dans un autre mode de réalisation, le domaine capable de se fixer au collagène peut également provenir du fragment du vWF compris entre les résidus 911 et 1114 et décrit par Pareti et al. [J. Biol. Chem. (1987) 262: 13835-13841]. La ligature de ces fragments avec le fragment de restriction HindIII-MstII correspondant à la totalité du gène codant pour la SAH à l'exception .des trois acides aminés les plus C-terminaux (cf. Figure 2) génère des fragments de restriction HindIII comportant un gène hybride codant pour une protéine chimère du type SAH-PEPTIDE (Figure 1, panneau A), immédiatement précédée de la région d'exportation "prépro" de la SAH. Ces fragments de restriction sont clones dans l'orientation productive dans le site HindIII du plasmide pYG105, ce qui génère les plasmides d'expression correspondants, et par exemple le plasmide pYG1277 (SAH-vWF509-695).

### E.7.3. Purification et caractérisation moléculaire des chimères entre SAH et vWF.

Les chimères présentes dans les surnageants de culture correspondant à la souche CBS 293.91 transformée, par exemple par les plasmides d'expression selon les exemples E.7.1. et E.7.2., sont caractérisées dans un premier temps à l'aide d'anticorps spécifiques de la partie SAH et de la partie vWF. Les résultats des Figures 5 à 7 démontrent que la levure K. lactis est capable de sécréter des protéines chimères entre la SAH et un fragment du vWF, et que ces chimères sont immunologiquement réactives. Il peut être également souhaitable de purifier certaines de ces chimères. La culture est alors centrifugée (10000 g, 30 min), le surnageant est passé à travers un filtre de 0,22 mm (Millipore), puis concentré par ultrafiltration (Amicon) en utilisant une membrane dont le seuil de discrimination se situe à 30 kDa. Le concentrat obtenu est alors dialysé contre une solution de Tris HCl (50 mM pH 8) puis purifié sur colonne. Par exemple, le concentrat correspondant au surnageant de culture de la souche CBS 293.91 transformée par le plasmide pYG1206 est purifiée par chromatographie d'affinité sur Bleu-Trisacryl (IBF). Une purification par chromatographie d'échange d'ions peut également être utilisée. Par exemple dans le cas de la chimère SAH-vWF470-713, le concentrat obtenu après ultrafiltration est dialysé contre une solution de Tris HCl (50 mM pH 8), puis déposé par fractions de 20 ml sur une colonne (5 ml) échangeuse de cations (S Fast Flow, Pharmacia) équilibrée dans le même tampon. La colonne est alors lavée plusieurs fois par la solution de Tris HCl (50 mM pH 8) et la protéine chimère est alors éluée de la colonne par un gradient (0 à 1 M) de NaCl. Les fractions contenant la protéine chimère sont alors réunies et dialysées contre une solution de Tris HCl 50 mM (pH 8) puis redéposées sur colonne S Fast Flow. Après élution de la colonne, les fractions contenant la protéine sont réunies, dialysées contre de l'eau et lyophilisées avant caractérisation: par exemple, le séquençage (Applied Biosystem) de la protéine [SAH-vWF470-704 C471G, C474G] sécrétée par la levure CBS 293.91 donne la séquence N-terminale attendue de la SAH (Asp-Ala-His...), démontrant une maturation correcte de la chimère immédiatement en C-terminal du doublet de résidus Arg-Arg de la région "pro" de la SAH (Figure 2). Le caractère essentiellement monomérique des protéines chimères entre SAH et vWF est également confirmé par leur profil d'élution sur colonne TSK 3000 [Toyo Soda Company, équilibrée par une solution de cacodylate (pH 7) contenant 0,2 M de Na₂SO₄] : par exemple la chimère [SAH-vWF 470-704 C471G, C474G]. se comporte dans ces conditions comme une protéine de poids moléculaire apparent de 95 kDa démontrant son caractère monomérique.

### EXEMPLE 8 : CHIMERES DERIVEES DE L'UROKINASE

### E.8.1. Constructions.

Un fragment correspondant au fragment amino-terminal de l'urokinase (ATF: domaine EGF-like + domaine kringle) peut être obtenu à partir de l'ARN messager correspondant des cellules de certains carcinome humain, par exemple en utilisant le kit RT-PCR distribué par Pharmacia. Un fragment de restriction MstII-HindIII incluant l'ATF de l'urokinase humaine est donné à la Figure 8. La ligature du fragment HindIII-MstII du plasmide pYG404 avec ce fragment MstII-HindIII permet de générer le fragment HindIII du plasmide pYG1341 qui code pour une protéine chimère dans laquelle la molécule de SAH est génétiquement couplée à l'ATF (SAH-UK1->135). De façon similaire, le plasmide pYG1340 contient un fragment HindIII codant pour une chimère composée de la SAH immédiatement suivi par les 46 premiers résidus de l'urokinase humaine (SAH-UK1->46. cf. Figure 8). Le clonage dans l'orientation productive du fragment de restriction HindIII du plasmide pYG1340 (SAH-UK1->46) dans le site HindIII des plasmides pYG105 (LAC4) et pYG106 (PGK) génère les plasmides d'expression pYG1343 et pYG1342, respectivement. De façon similaire, le clonage dans l'orientation productive du fragment de restriction HindIII du plasmide pYG1341 (SAH-UK1->135) dans le site HindIII des plasmides pYG105 (LAC4) et pYG106 (PGK) génère les plasmides d'expression pYG1345 et pYG1344, respectivement.

### E.8.2. Sécrétion des hybrides.

Après sélection sur milieu riche supplémenté en G418 les clones recombinants sont testés pour leur capacité à sécréter la forme mature des protéines chimères SAH-UK. Quelques clones correspondant à la souche K. lactis CBS 293.91 transformée par les plasmides d'expression selon l'exemple E.9.1. sont mis à incuber en milieu liquide complet sélectif à 28°C. Les surnageants cellulaires sont alors testés après électrophorèse en gel d'acrylamide à 8.5 %, soit directement par coloration du gel au bleu de coomassie, soit après immunoblot en utilisant comme. anticorps primaires un sérum polyclonal de lapin dirigé contre l'albumine humaine ou contre l'urokinase humaine. Les résultats de la Figure 9 démontrent que les protéines hybrides SAH-UK1->46 et SAH-UK1->135 sont particulièrement bien sécrétées par la levure Kluyveromyces.

### E.8.3. Purification des chimères entre SAH et urokinase.

Après centrifugation d'une culture de la souche CBS 293.91 transformée par les plasmides d'expression selon l'exemple E.8.1., le surnageant de culture est passé à travers un filtre de 0,22 mm (Millipore), puis concentré par ultrafiltration (Amicon) en utilisant une membrane dont le seuil de discrimination se situe à 30 kDa. Le concentrat obtenu est alors ajusté à 50 mM Tris HCl à partir d'une solution stock de Tris HCl 1M (pH 7), puis déposé par fractions de 20 ml sur une colonne (3 ml) échangeuse d'anions (D-Zephyr, Sepracor) équilibrée dans le même tampon. La protéine chimère (SAH-UK1->46 ou SAH-UK1->135) est alors éluée de la colonne par un gradient (0 à 1 M) de NaCl. Les fractions contenant la protéine chimère sont alors réunies et dialysées contre une solution de Tris HCl 50 mM (pH 6) et redéposées sur colonne D-Zephyr équilibrée dans le même tampon. Après élution de la colonne, les fractions contenant la protéine sont réunies, dialysées contre de l'eau et lyophilisées avant caractérisation de leur activité biologique et notamment vis à vis de leur aptitude à déplacer l'urokinase de son récepteur cellulaire.

### EXEMPLE 9 : CHIMERES DERIVEES DU G-CSF

### E.9.1. Constructions.

### E.9.1.1. Couplage en C-terminal de la SAH.

Un fragment de restriction MstII-HindIII incluant la forme mature du G-CSF humain est généré, par exemple selon la stratégie suivante : un fragment de restriction KpnI-HindIII est d'abord obtenu par la technique d'amplification enzymatique PCR en utilisant les oligodéoxynucléotides Sq2291 (5'-CAAGGATCCAAGCTTCAGGGCTGCGCAAGGTGGCGTAG-3', le site HindIII est souligné) et Sq2292 (5'-CGGGGTACCTTAGGCTTAACCCCCCTGGGCCCTGCCAGC-3', le site KpnI est souligné) comme amorce sur le plasmide BBG13 servant comme matrice. Le plasmide BBG13 comporte le gène.codant pour la forme B (174 acides aminés) du G-CSF mature humain, obtenu auprès de British Bio-technology Limited, Oxford, England. Le produit d'amplification enzymatique d'environ 550 nucléotides est ensuite digéré par les enzymes de restriction KpnI et HindIII et cloné dans le vecteur pUC19 coupé par les mêmes enzymes, ce qui génère le plasmide recombinant pYG1255. Ce plasmide est la source d'un fragment de restriction MstII-HindIII permettant de fusionner le G-CSF immédiatement en aval de la SAH (chimère SAH-G.CSF) et dont la séquence nucléotidique est donnée à la Figure 10.

Il peut être également souhaitable d'insérer un linker peptidique entre la partie SAH et G-CSF, par exemple pour permettre une meilleure présentation fonctionnelle de la partie transductrice. Un fragment de restriction MstII-HindIIIest par exemple généré par substitution du fragment MstII-ApaI du plasmide pYG1255 par les oligodéoxynucléotides Sq2742 (5'-TTAGGCTTAGGTGGTGGCGGTACCCCCCTGGGCC-3', les codons codant pour les résidus glycine de ce linker particulier sont soulignés) et Sq2741 (5'-CAGGGGGGTACCGCCACCACCTAAGCC-3') qui forment en s'appariant un fragment MstII-ApaI. Le plasmide ainsi généré comporte donc un fragment de restriction MstII-HindIII, dont la séquence est identique à celle de la Figure 10 à l'exception du fragment MstII-ApaI.

La ligature du fragment HindIII-MstII du plasmide pYG404 avec le fragment MstII-HindIII du plasmide pYG1255 permet de générer le fragment HindIII du plasmide pYG1259 qui code pour une protéine chimère dans laquelle la forme B du G-CSF mature est positionnée par couplage génétique en phase traductionnelle en C-terminal de la molécule de SAH (SAH-G.CSF).

Un fragment de restriction HindIII identique à l'exception du fragment MstII-ApaI peut également être facilement généré et qui code pour une protéine chimère dans laquelle la forme B du G-CSF mature est positionnée par couplage génétique en phase traductionnelle en C-terminal de la molécule de SAH et d'un linker peptidique particulier. Par exemple ce linker est constitué de 4 résidus glycine dans le fragment HindIII du plasmide pYG1336 (chimère SAH-Gly₄-G.CSF).

Le fragment de restriction HindIII du plasmide pYG1259 est cloné dans l'orientation productive et dans le site de restriction HindIII du plasmide d'expression pYG105, ce qui génère le plasmide d'expression pYG1266 (SAH-G.CSF). Dans une autre exemplification, le clonage du fragment de restriction HindIII du plasmide pYG1259 dans l'orientation productive et dans le site HindIII du plasmide pYG106 génère le plasmide pYG1267. Les plasmides pYG1266 et pYG1267 sont isogéniqùes entre eux à l'exception du fragment de restriction SalI-HindIII. codant pour le promoteur LAC4 de K. lactis (plasmide pYG1266) ou le promoteur PGK de S. cerevisiae (plasmide pYG1267).

Dans une autre exemplification, le clonage dans l'orientation productive du fragment de restriction HindIII du plasmide pYG1336 (chimère SAH-Gly4-G.CSF) dans le site HindIII des plasmides pYG105 (LAC4) et pYG106 (PGK) génère les plasmides d'expression pYG1351 et pYG1352, respectivement.

### E.9.1.2. Couplage en N-terminal de la SAH.

Dans un mode réalisation particulier, les techniques combinées de mutagénèse dirigée et d'amplification PCR permettent de construire des gènes hybrides codant pour une protéine chimère résultant du couplage traductionnel entre un peptide signal (et par exemple la région prépro de la SAH), une séquence incluant un gène ayant une activité G-CSF, et la forme mature de la SAH ou un de ses variants moléculaires (cf. chimère du panneau B, Figure 1). Ces gènes hybrides sont préférentiellement bordés en 5' de l'ATG initiateur de traduction et en 3' du codon de fin de traduction par des sites de restriction HindIII. Par exemple l'oligodéoxy-nucléotide Sq2369 (5'-GTTCTACGCCACCTTGCGCAGCCCGGTGGAGGCGGT-GATGCACACAAGAGTGAGGTTGCTCATCGG-3', les résidus soulignés (optionnels) correspondent dans cette chimère particulière à un linker peptidique composé de 4 résidus glycine) permet par mutagénèse dirigée de mettre en phase traductionelle la forme mature du G-CSF humain du plasmide BBG13 immédiatement en amont de la forme mature de la SAH, ce qui génère le plasmide intermédiaire A. De façon similaire, l'utilisation de l'oligodéoxynucléotide Sq2338 [5'-CAGGGAGCTGGCAGGGCCCAGGGGGGTTCGACGAAACACACCCCTGGAATAAGCCGAGCT-3' (brin non codant), les nucléotides complémentaires aux nucléotides codant pour les premiers résidus N-terminaux de la forme mature du G-CSF humain sont soulignés] permet par mutagénèse dirigée de coupler en phase traductionnelle de lecture la région prépro de la SAH immédiatement en amont de la forme mature du G-CSF humain, ce qui génère le plasmide intermédiaire B. On génère ensuite un fragment HindIII codant pour une protéine chimère du type PEPTIDE-SAH (cf. Figure 1, panneau B) en associant le fragment HindIII-SstI du plasmide B (jonction région prépro de la SAH + fragment N-terminal du G-CSF mature) avec le fragment SstI-HindIII du plasmide A [jonction G-CSF mature-(glycine)ₓ₄-SAH mature]. Le plasmide pYG1301 contient ce fragment de restriction HindIII particulier codant pour la chimère G.CSF-Gly4-SAH fusionnée immédiatement en aval de la région prépro de la SAH (Figure 11). Le clonage de ce fragment de restriction HindIII dans l'orientation productive et dans le site HindIII des plasmides pYG105 (LAC4) et pYG106 (PGK) génère les plasmides d'expression pYG1302 et pYG1303, respectivement.

### E.9.2. Sécrétion des hybrides.

Après sélection sur milieu riche supplémenté en G418 les clones recombinants sont testés pour leur capacité à sécréter la forme mature des protéines chimères entre SAH et G-CSF. Quelques clones correspondant à la souche K. lactis CBS 293.91 transformée par les plasmides pYG1266 ou pYG1267 (SAH-G.CSF), pYG1302 ou pYG1303 (G.CSF-Gly₄-SAH) ou encore pYG1351 ou pYG1352 (SAH-Gly₄-G.CSF) sont mis à incuber en milieu liquide complet sélectif à 28°C. Les surnageants cellulaires sont alors testés après électrophorèse en gel d'acrylamide à 8.5 %, soit directement par coloration du gel au bleu de coomassie, soit après immunoblot en utilisant comme anticorps primaires des anticorps polyclonaux de lapin dirigés contre le G-CSF humain ou un sérum polyclonal de lapin dirigé contre l'albumine humaine. Les résultats de la Figure 12 démontrent que la protéine hybride SAH-G.CSF est reconnue à la fois par des anticorps dirigés contre l'albumine humaine (panneau C) et le G-CSF humain (panneau B). Les résultats de la Figure 13 indiquent que la chimère SAH-Gly₄-G.CSF (piste 3) est particulièrement bien sécrétée par la levure Kluyveromyces, possiblement du fait que la présence du linker peptidique entre partie SAH et partie G-CSF est plus favorable à un repliement indépendant de ces 2 parties lors du transit de la chimère dans la voie sécrétoire. De plus la fusion N-terminale (G.CSF-Gly₄-SAH) est également sécrétée par la levure Kluvveromyces (Figure 13, piste 1).

### E.9.3. Purification et caractérisation moléculaire des chimères entre SAH et G-CSF.

Après centrifugation d'une culture de la souche CBS 293.91 transformée par les plasmides d'expression selon l'exemple E.9.1., le surnageant de culture est passé à travers un filtre de 0,22 mm (Millipore), puis concentré par ultrafiltration (Amicon) en utilisant une membrane dont le seuil de discrimination se situe à 30 kDa. Le concentrat obtenu est alors ajusté à 50 mM Tris HCl à partir d'une solution stock de Tris HCl 1M (pH 6), puis déposé par fractions de 20 ml sur une colonne (5 ml) échangeuse d'ions (Q Fast Flow, Pharmacia) équilibrée dans le même tampon. La protéine chimère est alors éluée de la colonne par un gradient (0 à 1 M) de NaCl. Les fractions contenant la protéine chimère sont alors réunies et dialysées contre une solution de Tris HCI 50 mM (pH 6) et redéposées sur colonne Q Fast Flow (1 ml) équilibrée dans le même tampon. Après élution de la colonne, les fractions contenant la protéine sont réunies, dialysées contre de l'eau et lyophilisées avant caractérisation: par exemple, le séquençage (Applied Biosystem) de la protéine SAH-G.CSF sécrétée par la levure CBS 293.91 donne la séquence N-terminale attendue de la SAH (Asp-Ala-His...), démontrant une maturation correcte de la chimère immédiatement en C-terminal du doublet de résidus Arg-Arg de la région "pro" de la SAH (Figure 2).

### EXEMPLE 10 : CHIMERES DERIVEES D'UNE IMMUNOGLOBULINE

### E.10.1. Constructions.

Un fragment Fv' peut être construit par les techniques du génie génétique, et qui code pour les fragments variables des chaines lourdes et légères d'une immunoglobuline (Ig), reliés entre eux par un peptide linker [Bird et al., Science (1988) 242: 423 ; Huston et al., (1988) Proc. Natl. Acad. Sci. 85: 5879]. Schématiquement, les régions variables (environ 120 résidus) des chaines lourdes et légères d'une Ig donnée sont clonées à partir de l'ARN messager de l'hybridome correspondant, par exemple en utilisant le kit RT-PCR distribué par Pharmacia (Mouse ScFv Module). Dans une seconde étape les régions variables sont génétiquement couplées par génie génétique par l'intermédiaire d'un peptide de liaison synthétique et par exemple le linker (GGGGS)ₓ₃. Un fragment de restriction MstII-HindIII incluant le fragment Fv' d'une immunoglobuline sécrétée par un hybridome murin est donné à la Figure 14. La ligature du fragment HindIII-MstII du plasmide pYG404 avec ce fragment MstII-HindIII permet de générer le fragment HindIII du plasmide pYG1382 qui code pour une protéine chimère dans laquelle la molécule de SAH est génétiquement couplée au fragment Fv' de la Figure 14 (chimère SAH-Fv'). Le clonage dans l'orientation productive du fragment de restriction HindIII du plasmide pYG1382 dans le site HindIII des plasmides pYG105 (LAG4) et pYG106 (PGK) génère les plasmides d'expression pYG1383 et pYG1384, respectivement.

### E.10.2. Sécrétion des hybrides.

Après sélection sur milieu riche supplémenté en G418 les clones recombinants sont testés pour leur capacité à sécréter la forme mature de la protéine chimère SAH-Fv'. Quelques clones correspondant à la souche K. lactis CBS 293.91 transformée par les plasmides pYG1383 ou pYG1384 (SAH-Fv') sont mis à incuber en milieu liquide complet sélectif à 28°C. Les surnageants cellulaires sont alors testés après électrophorèse en gel d'acrylamide à 8.5 %, soit directement par coloration du gel au bleu de coomassie, soit après immunoblot en utilisant comme anticorps primaires un sérum polyclonal de lapin dirigé contre l'albumine humaine, ou directement incubée avec des anticorps biotinylés et dirigés contre les immunoglobulines d'origine murine. Les résultats de la Figure 15 démontrent que la protéine hybride SAH-Fv' est reconnue à la fois par des anticorps dirigés contre l'albumine humaine (panneau C) et réagit avec des anticorps de chèvre biotinylés immunologiquement réactifs à l'encontre d'immunoglobulines de souris (panneau B).

### EXEMPLE 11 : ACTIVITE BIOLOGIQUE DES CHIMERES

### E.11.1. Activité biologique in vitro.

### E.11.1.1. Chimères entre SAH et vWF.

L'activité antagoniste des produits est déterminée par mesure de l'inhibition dose-dépendante de l'agglutination des plaquettes humaines fixées au paraformaldéhyde selon la méthode décrite par Prior et al. [Bio/Technology (1992) 10: 66]. Les mesures se font dans un agrégamètre (PAP-4, Bio Data, Horsham, PA, USA) qui enregistre les variations au cours du temps de la transmission optique sous agitation à 37°C en présence de vWF, de botrocétine (8,2 mg/ml) et du produit à tester à différentes dilutions (concentrations). Pour chaque mesure, 400 ml (8x10⁷ plaquettes) d'une suspension de plaquettes humaines stabilisées au paraformaldéhyde (0,5 %, puis resuspendues en [NaCl (137 mM) ; MgCl₂ (1 mM) ; NaH₂PO₄ (0,36 mM) ; NaHCO₃ (10 mM) ; KCl (2,7 mM) ; glucose (5,6 mM) ; SAH (3,5 mg/ml) ; tampon HEPES (10 mM, pH 7,35)] sont préincubés à 37°C dans la cuve cylindrique (8,75 x 50 mm, Wellcome Distriwell, 159 rue Nationale, Paris) de l'agrégamètre pendant 4 min puis sont additionnés de 30 ml de la solution du produit à tester à différentes dilutions dans du véhicule de formulation apyrogène [mannitol (50 g/l) ; acide citrique (192 mg/l) ; L-lysine monochlorhydratée (182,6 mg/l). ; NaCl (88 mg/l) ; pH ajusté à 3,5 par addition de NaOH (1M)], ou de véhicule de formulation uniquement (essai contrôle). La suspension résultante est alors incubée pendant 1 min à 37°C et on ajoute 12,5 ml de vWF humain [American Bioproducts, Parsippany, NJ, USA; 11 % d'activité von Willebrand mesurée selon les recommandations d'utilisation du PAP-4 (Platelet Aggregation Profiler^{R}) à l'aide de plaquettes fixées au formaldéhyde (2x10⁵ plaquettes/ml), de plasma humain contenant de 0 à 100 % de vWF et de ristocétine (10 mg/ml, cf. p. 36-45 : vW Program™] que l'on incube à 37°C pendant 1 min avant d'ajouter 12,5 ml de la solution de botrocétine [purifiée à partir de venin lyophilisé de Bothrops jararaca (Sigma), selon le protocole décrit par Sugimoto et al., Biochemistry (1991) 266: 18172]. L'enregistrement de la lecture de la transmission en fonction du temps est alors réalisée pendant 2 min sous agitation à l'aide d'un barreau aimanté (Wellcome Distriwell) placé dans la cuve et sous une agitation magnétique de 1100 tr/min assurée par l'agrégamètre. La variation moyenne de la transmission optique (n³5 pour chaque dilution) au cours du temps est donc une mesure de l'agglutination plaquettaire due à la présence de vWF et de botrocétine, en l'absence ou en présence de concentrations variables du produit à tester. A partir de tels enregistrements, on détermine alors le % d'inhibition de l'agglutination plaquettaire due à chaque concentration de produit et on trace la droite donnant le % d'inhibition en fonction de l'inverse de la dilution de produit en échelle log-log. La CI50 (ou concentration de produit provoquant 50 % d'inhibition de l'agglutination) est alors déterminée sur cette droite. Le Tableau de la Figure 16 compare les CI50 de quelques unes des chimères SAH-vWF de la présente invention et démontre que certaines d'entre elles sont de meilleurs antagonistes de l'agglutination plaquettaire que le produit RG12986 décrit par Prior et al. [Bio/Technology (1992) 10: 66] et inclus dans les essais à titre de valeur étalon. Des tests identiques de l'inhibition de l'agglutination de plaquettes humaines en présence de vWF de plasma de porc (Sigma) permet en plus de démontrer que certains des hybrides de la présente invention, et notamment certains variants de type IIB, sont de très bons antagonistes de l'agglutination plaquettaire en l'absence de co-facteurs de type botrocétine. L'antagonisme botrocétine-indépendant de ces chimères particulières peut également être démontré selon le protocole initialement décrit par Ware et al. [Proc. Natl. Acad. Sci. (1991) 88: 2946] par déplacement de l'anticorps monoclonal ¹²⁵I-LJ-IB1 (10 mg/ml), un inhibiteur compétitif de la fixation du vWF sur la GPIb plaquettaire [Handa M. et al., (1986) J. Biol. Chem. 261: 12579] après 30 min d'incubation à 22°C en présence de plaquettes fraiches (10⁸ plaquettes/ml).

### E.11.1.2. Chimères entre SAH et G-CSF.

Les chimères purifiées sont testées pour leur capacité à permettre la prolifération in vitro de la lignée murine IL3-dépendante NFS60, par mesure de l'incorporation de thymidine tritiée essentiellement selon le protocole décrit par Tsuchiya et al. [Proc. Natl. Acad. Sci. (1986) 83 7633]. Pour chaque chimère, les mesures sont réalisées entre 3 et 6 fois dans un test trois points (trois dilutions du produit) dans une zone ou la relation entre quantité de produit actif et incorporation de thymidine marquée (Amersham) est linéaire. Dans chaque plaque de microtitration, l'activité d'un produit référence constitué de G-CSF humain recombinant exprimé dans des cellules mammifères est également systématiquement incorporé. Les résultats de la Figure 17 démontrent que la chimère SAH-G.CSF (pYG1266) sécrétée par la levure Kluyveromyces et purifiée selon l'exemple E.9.3. est capable in vitro de transduire un signal de prolifération cellulaire pour la lignée NFS60. Dans ce cas particulier, l'activité spécifique (cpm/molarité) de la chimère est environ 7 fois plus faible que celle du G-CSF référence (non couplé).

### E.11.2. Activité biologique in vivo.

L'activité de stimulation des chimères SAH/G-CSF sur la granulopoièse in vivo est testée après injection sous-cutanée chez le rat (Sprague-Dawley/CD, 250-300 g, 8-9 semaines) et comparée à celle du G-CSF référence exprimé à partir de cellules de mammifère. Chaque produit, testé à raison de 7 animaux, est injecté par voie sous-cutanée en région dorso-scapulaire à raison de 100 ml pendant 7 jours consécutifs (J1-J7). 500 ml de sang sont receuillis aux jours J-6, J2 (avant la 2^{ème} injection), J5 (avant la 5^{ème} injection) et J8, et une numération sanguine est effectuée. Dans ce test, l'activité spécifique (unités de neutropoièse/mole injectée) de la chimère SAH-G.CSF (pYG1266) est identique à celle du G-CSF référence (Figure 18). Puisque cette chimère particulière possède in vitro une activité spécifique 7 fois plus faible que celle du G-CSF référence (Figure 17), il est donc démontré que le couplage génétique du G-CSF sur la SAH en modifie favorablement les propriétés pharmacocinétiques.

LISTE DE SEQUENCES

(2) INFORMATION POUR LA SEQ ID NO: 1:

(i) CARACTERISTIQUES DE LA SEQUENCE:
(A) LONGUEUR: 1859 paires de bases
(B) TYPE: acide nucléique
(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(ix) CARACTERISTIQUE ADDITIONELLE:
(A) NOM/CLE: CDS
(B) EMPLACEMENT: 26..1855
(D) AUTRES RENSEIGNEMENTS: /product= "Chimere de type
SAH-Peptide"

(ix) CARACTERISTIQUE ADDITIONELLE:
(A) NOM/CLE: misc_feature
(B) EMPLACEMENT: 1842..1848
(D) AUTRES RENSEIGNEMENTS: /standard_name= "Site Mst II"

(2) INFORMATION POUR LA SEQ ID NO: 2:

(i) CARACTERISTIQUES DE LA SEQUENCE:
(A) LONGUEUR: 750 paires de bases
(B) TYPE: acide nucléique
(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(ix) CARACTERISTIQUE ADDITIONELLE:
(A) NOM/CLE: CDS
(B) EMPLACEMENT: 3..746
(D) AUTRES RENSEIGNEMENTS: /product= "Fragment C-ter de la
chimere SAH-vWF470"

(2) INFORMATION POUR LA SEQ ID NO: 3:

(i) CARACTERISTIQUES DE LA SEQUENCE:
(A) LONGUEUR: 423 paires de bases
(B) TYPE: acide nucléique
(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(ix) CARACTERISTIQUE ADDITIONELLE:
(A) NOM/CLE: CDS
(B) EMPLACEMENT: 3..419
(D) AUTRES RENSEIGNEMENTS: /product= "Fragment C-ter de la
chimere SAH-UK1-135"

(2) INFORMATION POUR LA SEQ ID NO: 4:

(i) CARACTERISTIQUES DE LA SEQUENCE:
(A) LONGUEUR: 541 paires de bases
(B) TYPE: acide nucléique
(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(ix) CARACTERISTIQUE ADDITIONELLE:
(A) NOM/CLE: CDS
(B) EMPLACEMENT: 3..536
(D) AUTRES RENSEIGNEMENTS: /product= "Fragment C-ter de la
chimere SAH-G.CSF"

(2) INFORMATION POUR LA SEQ ID NO: 5:

(i) CARACTERISTIQUES DE LA SEQUENCE:
(A) LONGUEUR: 2455 paires de bases
(B) TYPE: acide nucléique
(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(ix) CARACTERISTIQUE ADDITIONELLE:
(A) NOM/CLE: CDS
(B) EMPLACEMENT: 26..2389
(D) AUTRES RENSEIGNEMENTS: /product= "Chimere G.CSF-Gly4-SAH
en aval region prepro de SAH"

(ix) CARACTERISTIQUE ADDITIONELLE:
(A) NOM/CLE: misc_recomb
(B) EMPLACEMENT: 620..631
(D) AUTRES RENSEIGNEMENTS: /standard_name= "Linker PolyGly"

(ix) CARACTERISTIQUE ADDITIONELLE:
(A) NOM/CLE: misc_feature
(B) EMPLACEMENT: 106..111
(D) AUTRES RENSEIGNEMENTS: /standard_name= "Site Apa I"

(2) INFORMATION POUR LA SEQ ID NO: 6:

(i) CARACTERISTIQUES DE LA SEQUENCE:
(A) LONGUEUR: 756 paires de bases
(B) TYPE: acide nucléique

(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(ix) CARACTERISTIQUE ADDITIONELLE:
(A) NOM/CLE: CDS
(B) EMPLACEMENT: 3..752
(D) AUTRES RENSEIGNEMENTS: /product= "Fragment C-ter de la
chimere SAH-Fv'"

(ix) CARACTERISTIQUE ADDITIONELLE:
(A) NOM/CLE: misc_recomb
(B) EMPLACEMENT: 384..428
(D) AUTRES RENSEIGNEMENTS: /standard_name= "Linker
synthetique"

## Revendications

1. Polypeptide recombinant comportant une partie active dérivée d'un polypeptide ayant une activité thérapeutique, génétiquement couplée à une albumine ou à un variant de l'albumine.

2. Polypeptide selon la revendication 1 **caractérisé en ce que** le polypeptide ayant une activité thérapeutique est un polypeptide d'origine humaine.

3. Polypeptide selon la revendication 2 **caractérisé en ce que** le polypeptide ayant une activité thérapeutique est choisi parmi tout ou partie des enzymes, des inhibiteurs d'enzymes, des antigènes, des anticorps, des hormones, des facteurs de la coagulation, des interférons, des cytokines, des facteurs de croissance et/ou de différenciation, des facteurs impliqués dans la génèse/résorption des tissus osseux, des facteurs chimiotactiques, des facteurs de motilité ou de migration cellulaire, des facteurs cytostatiques, des facteurs bactéricides ou antifongiques, ou des molécules adhésives plasmatiques, interstitielles ou des matrices extracellulaires.

4. Polypeptide selon l'une des revendications 1 à 3 **caractérisé en ce que** le polypeptide ayant une activité thérapeutique est choisi parmi toute séquence peptidique antagoniste ou agoniste d'interactions moléculaires et/ou cellulaires impliquées dans les pathologies des compartiments circulatoires et interstitiels.

5. Polypeptide selon l'une des revendications 1 à 4 **caractérisé en ce que** la partie active présente une structure choisie parmi :
(a) la structure peptidique entière ou,
(b) un fragment de (a) ou une structure dérivée de (a) par modification structurale (mutation, substitution addition et/ou délétion d'un ou plusieurs résidus) et conservant une activité thérapeutique.

6. Polypeptide selon l'une des revendications 1 à 5 **caractérisé en ce que** la partie active est couplée à l'extrémité N-terminale de l'albumine.

7. Polypeptide selon l'une des revendications 1 à 5 **caractérisé en ce que** la partie active est couplée à l'extrémité C-terminale de l'albumine.

8. Polypeptide selon l'une des revendications 1 à 7 **caractérisé en ce que** la partie active y est représenté plusieurs fois.

9. Séquence nucléotidique codant pour un polypeptide selon l'une quelconque des revendications 1 à 8.

10. Séquence nucléotidique selon la revendication 9 **caractérisée en ce qu'**elle comprend une séquence "leader" permettant la sécrétion du polypeptide exprimé.

11. Cassette d'expression comprenant une séquence nucléotidique selon l'une des revendications 9 ou 10 sous le contrôle d'une région d'initiation de la transcription et éventuellement d'une région de terminaison de la transcription.

12. Plasmide autoréplicatif comportant une cassette d'expression selon la revendication 11.

13. Cellule recombinante eucaryote ou procaryote dans laquelle a été inséré une séquence nucléotidique selon l'une des revendications 9 ou 10 ou une cassette d'expression selon la revendication 11 ou un plasmide selon la revendication 12.

14. Cellule recombinante selon la revendication 13 **caractérisée en ce qu'**il s'agit d'une levure, d'une cellule animale, d'un champignon ou d'une bactérie.

15. Cellule recombinante selon la revendication 14 **caractérisée en ce qu'**il s'agit d'une levure.

16. Cellule recombinante selon la revendication 15 **caractérisée en ce qu'**il s'agit d'une levure du genre Saccharomyces ou Kluyveromyces.

17. Procédé de préparation d'un polypeptide tel que défini dans l'une des revendications 1 à 8 **caractérisé en ce que** l'on cultive une cellule recombinante selon l'une des revendications 13 à 16 dans des conditions d'expression, et on récupère le polypeptide produit.

18. Composition pharmaceutique comprenant un ou plusieurs polypeptides selon l'une quelconque des revendications 1 à 8.

19. Composition pharmaceutique comprenant une séquence nucléotidique selon l'une quelconque des revendications 9 à 11 utilisable en thérapie génique.
